# EUROPÄISCHE PATENTSCHRIFT

(11) **EP 2 440 287 B1**
(45) Veröffentlichungstag und Bekanntmachung des Hinweises auf die Patenterteilung: **24.07.2013**
(21) Anmeldenummer: 10744674.2
(22) Anmeldetag: 09.06.2010
(51) Int. Cl.: A61N 5/06, A61B 19/00

(54) **VORRICHTUNG FÜR DIE PHOTODYNAMISCHE THERAPIE**
DEVICE FOR PHOTODYNAMIC THERAPY
DISPOSITIF DE PHOTOCHIMIOTHÉRAPIE

(30) Priorität: 09.06.2009 DE 102009024860
(43) Veröffentlichungstag der Anmeldung: 18.04.2012
(73) Patentinhaber: bredent medical GmbH & Co. KG, 89250 Senden (DE)
(72) Erfinder: VIZETHUM, Freimut, 69231 Rauenberg (DE); SCHÜTZE, Reinhold, A-4800 Attnang-Puchheim (AT)
(74) Vertreter: Schmitt, Meinrad
(86) Internationale Anmeldenummer: PCT/EP2010/003457
(87) Internationale Veröffentlichungsnummer: WO 2010/142430

(56) Entgegenhaltungen:
- US-A1- 2004 008 523
- US-A1- 2008 147 053

## Beschreibung

Die Erfindung bezieht sich auf eine Vorrichtung für die photodynamische Therapie und / oder zur Abtötung oder Reduktion von Mikroorganismen, gemäß den im Oberbegriff des Patentanspruchs 1 angegebenen Merkmalen.

Aus der US 2004/0008523 A1 sind eine derartige Vorrichtung und deren Verwendung bekannt, wobei die Vorrichtung eine Bestrahlungseinheit mit Lichtquellen, eine Kamera zur Erfassung von Bildern einer Wunde und ferner eine Positioniereinrichtung vorgesehen ist, mittels welcher die Bestrahlungseinheit zur Wunde ausrichtbar ist. Ferner ist ein Display zur Darstellung des Kamerabilds und eines überlagerten eingeblendeten Rasters sowie Markierungsmitteln vorhanden. Auf dem Display werden nebeneinander Bilder von zu verschiedenen Zeiten aufgenommenen Wundarealen dargestellt, um den Heilungsprozess bestimmen zu können. Das Raster und ebenso die Markierungen ermöglichen die exakte Ausrichtung der Kamera derart, dass die Kamera für beide Bilder jeweils auf den gleichen Zielbereich gerichtet ist. Mit der Positioniereinrichtung ist die Bestrahlungseinheit insgesamt zum Wundareal einstellbar, wobei eine Bewegung oder Führung der Lichtquellen auf oder in der Bestrahlungseinheit nicht vorgesehen ist.

Aus der WO 2005/035058 A1 sind eine Vorrichtung und ein Verfahren bekannt, welche eine mittels einer Positioniereinrichtung bewegbare Bestrahlungseinheit aufweisen. Die Bestrahlungseinheit enthält mehrere Lichtquellen, mittels welchen ein auf das zu therapierende Wundareal aufgebrachte Photosensitizer aktiviert wird. Die Bestrahlungseinheit enthält eine Kamera, mittels welcher vor Durchführung und während der Durchführung der Therapie Aufnahmen vom Wundareal gemacht werden. Die Lichtquellen sind in Form einer Cluster-Lampe mit einer Anzahl von lichtemittierenden Dioden (LED) ausgebildet und in der Bestrahlungseinheit mittels eines Kühlkörpers festgelegt. Weiterhin sind Abstandssensoren zur Überwachung des Abstands der Bestrahlungseinheit zum Wundareal vorgesehen.

Ferner ist aus der WO 2004/105 874 A2 eine Vorrichtung bekannt, welche eine Bestrahlungseinheit mit einer insbesondere als Laser ausgebildeten Lichtquelle enthält. Mit Hilfe einer lichtaktivierbaren Substanz, insbesondere eines Farbstoffes werden Mikroorganismen sensibilisiert und / oder angefärbt und nach Bestrahlung mit Licht geeigneter Wellenlänge und Energiedichte abgetötet. Die Wirkungsprinzip der Photodynamischen Therapie (PDT) bzw. der antimikrobiellen Photodynamischen Therapie (aPDT) beruht nach einer selektiven Einwirkung und / oder Anfärbung der Mikroorganismen auf der physikalischen Einwirkung der Energieübertragung auf eine lichtaktivierbare Substanz, welche auch als Photosensitizer oder Photosensibilisator bezeichnet wird, wobei die Energie für Reaktionen an der Zellmembran zur Verfügung gestellt wird. Die mittels der Lichtquelle der Bestrahlungseinheit erzeugte Energie wird dabei auf die Mikroorganismen konzentriert und die Gleichgewichtslage von Reaktionen, welche im nichtbelichteten Zustand im "normalen Milieu" ablaufen, werden verschoben und infolgedessen werden die Mikroorganismen zerstört. Die vorbekannte Vorrichtung enthält einen mit der Bestrahlungseinheit koppelbaren Applikator mit einem Lichtleiter, wobei das freie Ende des Applikators möglichst nahe an den zu therapierenden Bereich zwecks Bestrahlung desselben herangeführt wird. Diese Vorrichtung hat sich besonders in der Zahnmedizin bzw. im Mund-, Kiefer- oder Gesichtsbereich bewährt. Zur Therapie z.B. großflächiger Wunden oder für den Bereich der Wundheilung ist die bekannte Vorrichtung nicht ohne weiteres einsetzbar. Es sei hier nur beispielshaft auf typische chronische Wunden bzw. Hautgeschwüre verwiesen, wie Wundgeschwüre, welche bei einem nicht mehr mobilen Patienten in der Steißbeinregion auftreten (Decubitus), Unterschenkelgeschwüre auf der Basis von Krampfadern bzw. Gefäßverschlüssen (z.B. Ulcus cruris), Hautgeschwüre, welche durch Zuckerkrankheit entstehen können, z.B. diabetisches Fußsyndrom (Fuß-Ulcus) oder akut infizierte Wunden wie z.B. OP-Wunden.

Hiervon ausgehend liegt der Erfindung die Aufgabe zugrunde, eine Vorrichtung und ein Verfahren für die Anwendung der photodynamischen Therapie (PDT) bzw. antimikrobiellen Photodynamische Therapie(aPDT) in der Wundheilung vorzuschlagen, wobei insbesondere eine zuverlässige Anwendung und / oder nachgewiesene Keimabtötung für eine möglichst große Wundfläche in kurzer Zeit erreicht wird. Es soll eine effektive und nach den jeweiligen Gegebenheiten steuerbare Wundtherapie mit einem möglichst geringen apparativen Aufwand und / oder bei einfacher Handhabung und / oder mit hoher Funktionssicherheit erreicht werden. Die Vorrichtung soll ebenso wie das Verfahren problemlos entsprechend den medizinischen und / oder therapeutischen Anforderungen anpassbar sein. Die Vorrichtung soll sich in einfacher Weise und ohne großen Aufwand an die verschiedenen Positionen von Wunden anpassen lassen, wobei eine einfache Verstellmöglichkeit gegeben sein soll. Ferner soll das Gewicht der Bestrahlungseinheit möglichst gering vorgegeben werden können, damit sie von der Positioniereinrichtung zuverlässig in einer vom Behandler eingestellten Position gehalten werden kann. Die Beweglichkeit und / oder Mobilität der Vorrichtung soll optimiert werden, wobei vor allem ein geringes Gewicht und / oder kleine Abmessungen erreicht werden sollen. Ferner soll eine erhöhte Dämpfung hinsichtlich Stößen oder Schlägen erreicht werden, um Beschädigungen der Lichtquelle und / oder Laserdioden zu vermeiden.

Die Lösung dieser Aufgabe erfolgt hinsichtlich der Vorrichtung gemäß den im Patentanspruch 1 angegebenen Merkmalen.

Die erfindungsgemäße Vorrichtung ermöglicht bei einfachem Aufbau und / oder einfacher Handhabung eine funktionssichere und praxisgerechte Anwendung im Bereich der Wundheilung. Das erfindungsgemäße Therapiesystem für die Anwendung der PDT bzw. aPDT in der Wundheilung ermöglicht eine zuverlässige Anwendung und / oder eine nachgewiesene optimale Keimabtötung in kurzer Zeit auch und gerade für eine möglichst große Wundfläche. Ein im Photosensitizer enthaltener, insbesondere blauer Farbstoff und / oder HELBO Blue Kutan, wird auf den Therapiebereich und / oder die zu behandelnde Wunde bzw. Teile davon aufgetragen. Für das Einwirken des Photosensitizers wird eine definierte Zeit vorgegeben, insbesondere wenigstens 2 min., derart, dass eine Bindung der Farbstoffmoleküle an die Mikroorganismen ermöglicht wird. Nachfolgend wird zweckmäßig überschüssiger Farbstoff abgespült und / oder abgetupft, wobei bevorzugt folgendes Prozedere angewendet wird: Absaugen des überschüssigen Farbstoffs mit einem Stieltupfer, Überstreichen des Therapieareals mit einem mit physiologischer Kochsalzlösung, NaCl-getränktem Tupfer und schließlich Absaugen der Restflüssigkeit mit weiterem Stieltupfer. Daraufhin erfolgt die Belichtung mit lichtgeeigneter Wellenlänge und Energie. Hierzu wird bevorzugt Licht mit einer Wellenlänge in der Größenordnung von 661 nm, einer Leistungsdichte von mindestens 100 mW/cm² und mit einer Energie von zumindest näherungsweise 3-5 J/cm² vorgesehen und / oder derart, dass der Photosensitizer aktiviert und somit die Abtötung der Mikroorganismen induziert wird.

Die wenigstens eine Lichtquelle ist in der Bestrahlungseinheit mittels einer Führungseinrichtung, welche insbesondere als Linearführung ausgebildet ist, bewegbar angeordnet, um auf wenigstens zwei unterschiedliche Bestrahlungspositionen des Wundareals ausgerichtet zu werden. Das mittels der Kamera erfasste Wundareal wird auf einem Display zur Anzeige gebracht und ferner wird in dem Display ein Raster zur Anzeige gebracht bzw. dem eingeblendeten Kamera-bild des Wundareals überlagert, wobei in bevorzugter Weise jedes Rasterfeld einem Bestrahlungsfeld entspricht, insbesondere entsprechend der jeweiligen Positionierung der wenigstens einen in der Bestrahlungseinheit mittels der Führungseinrichtung sequentiell und / oder nacheinander von Bestrahlungsfeld zum nächsten Bestrahlungsfeld bewegbar angeordneten Lichtquelle. Von einem Behandler können diejenigen Felder ausgewählt und / oder markiert werden, welche der mittels wenigstens einen Lichtquelle zu bestrahlen sind. Das Display ist vorteilhaft Bestandteil einer Bedieneinheit, welche zusätzliche Tasten zum Scannen bzw. Erzeugen des Bildes des Wundareals sowie zum Start oder Stopp der Bestrahlung oder sonstige Eingabetasten enthält. Des weiteren enthält die Bedieneinheit in bevorzugter Weise Anzeigelemente für den mittels Abstandssensoren erfassten Abstand der Bestrahlungseinheit zu der zu therapierenden Wunde bzw. des zu therapierenden Körperteils. Das Display ist in bevorzugter Weise zusammen mit den Tasten und der Abstandsanzeige in einer Bedieneinheit integriert, welche zweckmäßig außen auf der Bestrahlungseinheit angeordnet ist und / oder als separate für einen Benutzer gut erreichbare Einheit ausgebildet ist.

Mittels der erfindungsgemäßen Vorrichtung und / oder dem Therapiesystem werden vor allem folgende Vorteile erzielt:
- synergistischer Einsatz mit den derzeit vorhandenen Möglichkeiten mit zumindest additiv erhöhter Wirkung,
- Reduzierung der Kosten der Wundbehandlung insgesamt, da teure, oft zu wechselnde, Verbandsstoffe eingespart werden können,
- eine merklich erhöhte Effizienz, insbesondere der Keimabtötung,
- Nachhaltigkeit durch Einsparung von teuren und limitierten Ressourcen, wie z.B. Silber in bekannten Verbandsstoffen,
- fehlende Resistenzentwicklung wie bei der klassischen systemischen Antibiotikatherapie,
- Vermeidung von Nebenwirkungen der derzeit verwendeten Therapieformen, wie Gefahr der Auslösung von Allergien oder Schmerzsymptomatik durch Silber,
- schnellere Wundheilung, wodurch kürzere Klinikaufenthalte vor allem bei chronischen Wundheilungsstörungen erreicht werden,
- Einsparung bei Medikamentenkosten und Verbandsmaterial,
- insgesamt wirtschaftliche Vorteile, insbesondere in Form von Kosteneinsparungen, für die Träger der Gesundheitseinrichtungen und folglich für das gesamte Gesundheitssystem.

Die erfindungsgemäße Vorrichtung und / oder das Bestrahlungsgerät bestehen im Wesentlichen aus folgenden Komponenten bzw. Modulen, welche weiter unten näher erläutert werden:
- Bestrahlungseinheit (Bestrahlungskopf) incl. einer elektronischen oder mechanischen Abstandsüberwachung, wobei optional oder bevorzugt eine Bedieneinheit enthalten ist,
- Energieversorgungseinheit, welche optional in einen Gerätewagen integriert ist, incl. Sicherheiteinrichtungen,
- Gelenkarm und / oder Positionierarm zur Verbindung der Bestrahlungseinheit mit einem Gerätewagen,
- Gerätewagen, welcher die gesamte Einheit trägt und / oder aufnimmt.

Die erfindungsgemäße Vorrichtung sowie das auf deren Verwendung gerichtete Verfahren ermöglichen bei einfacher Handhabung und mit hoher Funktionssicherheit die Durchführung der PDT zur Reduktion pathogener Keime (aPDT), insbesondere für die Behandlung folgender Hautwunden:
- chronische Wunden (Ulcus Decubitus)
- akut infizierte Wunden (z.B. Post-OP)
- Wunden mit lokalen Infektionen
- sowie Induzierung der Wundheilung bei stagnierenden Wunden.

Hierbei gelangen folgende Komponenten oder Module zum Einsatz:
- Lichtquelle / Bestrahlungsgerät
- Photosensitizerlösungen, insbesondere HELBO Blue Kutan
- Hilfsmittel wie Applikatoren oder Tupfer.

Die Eleminierung - u.a. - der im folgenden angeführten pathogenen Keime ist indiziert und wurde durch eine klinische Pilotstudie nachgewiesen. Ein typisches Keimspektrum in den erfindungsgemäß behandelten Wunden umfasst folgende Erreger:
- Staphylococcus aureus - Bacteroides species
- Escherichia coli - Proteus species
- Enterococcus spp - Streptococcus Gruppe F
- Vergrün. Streptokokken - Staphylococcus schleiferi
- Streptococcus agalactiae (B) - Enterobacter cloacae
- Coagulase neg. Staph. - Streptococcus Pyogenes
- Pseudomonas aeruginosa

Das erfindungsgemäße Therapiesystem, welches auch als HELBO Wounds bezeichnet wird, enthält im Wesentlichen folgende Komponenten:
- Farbstoff (Photosensitizer)
- Bestrahlungsgerät sowie
- optionale Hilfsmittel, wie z.B. in Form eines sterilen Therapiesets, welches den Farbstoff sowie Applikatoren, Abtupfer, Pinsel etc. enthält.

Als Photosensitizer wird insbesondere der bereits zugelassene Farbstoff HELBO Blue Kutan verwendet. Dieser Farbstoff ist ein Verbrauchsmaterial und wird in Einzeldosen abgepackt, welche für eine Wundbehandlung mit definierter maximaler Wundfläche ausreicht.

Die Erfindung wird nachfolgend anhand der in der Zeichnung dargestellten Ausführungsbeispiele näher erläutert, ohne dass insoweit eine Beschränkung erfolgt. Es zeigen in schematischen Darstellungen:
- Fig. 1: den Aufbau des Gesamtsystems,
- Fig. 2: eine bewegbar angeordnete Lichtquelle,
- Fig. 3: eine Ausbildung der Lichtquelle zur räumlichen Bestrahlung,
- Fig. 4 - 6: Ansichten der Bestrahlungseinheit mit Gehäuse und Gelenkarm,
- Fig. 7: Anordnung von drei Lasereinheiten der Bestrahlungseinheit bzw. des Bestrahlungsgeräts.
- Fig. 8: eine Ansicht der Bedieneinheit,
- Fig. 9: eine alternative Ausgestaltung der Bedieneinheit,
- Fig. 10: Positionierung der Bestrahlungseinheit über eine Wunde an einem Unterschenkel,
- Fig. 11: ein Flussdiagramm zur Positionierung der Bestrahlungseinheit,
- Fig. 12 - 14: Darstellung einer ebenen und einer räumlichen Bestrahlung,
- Fig. 15: ein Flussdiagramm zur Positionierung der schwenkbaren Lichtquelle bzw. Laserdiode,
- Fig. 16: ein Kamerabild einer Wunde mit eingeblendetem Raster im Display,
- Fig. 17: ein Kamerabild entsprechend Fig. 16 mit markierten ausgewählten Bestrahlungsfeldern,
- Fig. 18: ein Flussdiagramm zur Bestrahlung incl. kontinuierlicher Abstandskontrolle,
- Fig. 19, 20: Kamerabilder der Wunde im Display mit Raster und ausgewählten Feldern in verschiedenen Bestrahlungspositionen.

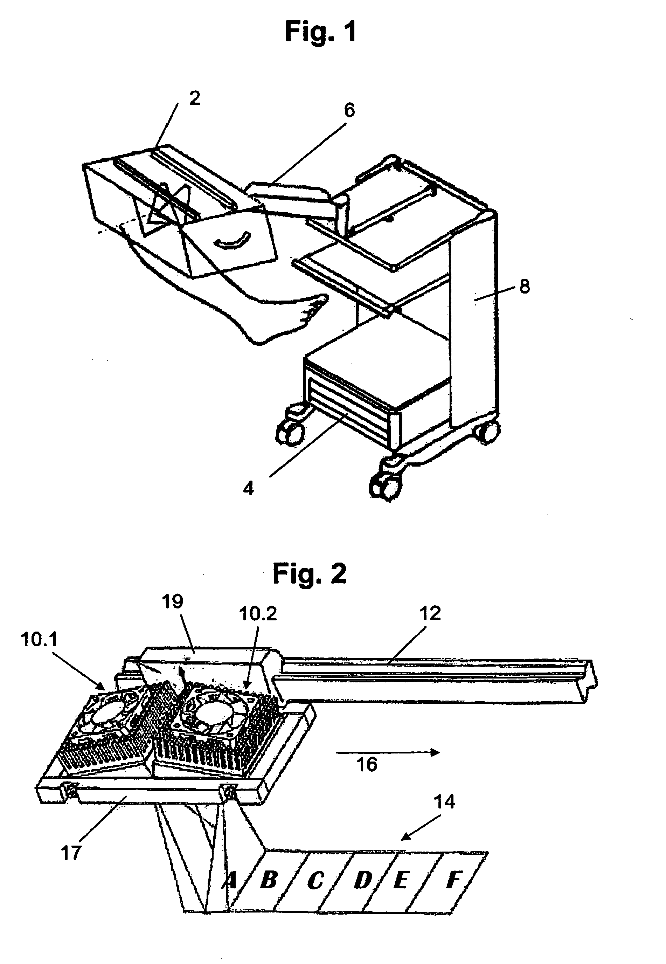

Fig. 1 zeigt den Aufbau des Gesamtsystems oder Therapiesystems, welches auch als HELBO Wounds bezeichnet wird und im Wesentlichen folgende Komponenten enthält:
- Die zum Einsatz gelangenden Lichtquelle und / oder Lichtquellen, insbesondere für Laserlicht, können eine vorgegebene Fläche mit der geforderten Leistungsdichte bestrahlen,
- Um innerhalb vorgegebener, bevorzugt kurzer Zeit, mit einer geringen Anzahl von Lichtquellen, insbesondere Laserdioden, die Gesamtfläche des Therapieareals beleuchten zu können, werden in der Bestrahlungseinheit mittels einer Führungseinrichtung und einer Antriebseinheit die Lichtquelle und / oder Lichtquellen erfindungsgemäß von einer Bestrahlungsposition zur nächsten bewegt, wobei jeweils Teilflächen bestrahlt werden.

Das Bestrahlungsgerät und / oder die Vorrichtung bestehen im Wesentlichen aus folgenden Komponenten, deren detaillierte Eigenschaften weiter unten noch erläutert werden:
- Bestrahlungseinheit 2 (Bestrahlungskopf) incl. Abstandsüberwachung, welche elektronisch oder mechanisch erfolgt und / oder ausgebildet ist, wobei bevorzugt ferner eine Bedieneinheit, welche insbesondere ein Display aufweist, integriert ist,
- Bedieneinheit,
- Energieversorgungseinheit 4, welche bevorzugt in einen Gerätewagen 8 integriert ist und ferner bevorzugt Sicherheitseinrichtungen enthält,
- Gelenkarm 6 und / oder Positionierarm und / oder Positioniereinrichtung, zur Verbindung von Bestrahlungseinheit 2 und Gerätewagen 8,
- Gerätewagen 8, welcher die gesamte Einheit trägt.

Die Anwendung des HELBO Wounds Systems und / oder die Durchführung des Verfahrens ist nach der Wundreinigung und vor dem Applizieren einer Wundauflage vorgesehen. Alternativ kann die Anwendung und / oder Durchführung des Verfahrens abhängig von Anwendungsstudien und Anwendungsbeobachtungen erfolgen.

Zentraler Bestandteil der Vorrichtung und des Verfahrens ist die Ausbildung des Bestrahlungskopfes und / oder der Bestrahlungseinheit 2, insbesondere in Lasertechnologie. Um die geforderten Leistungsdaten zu erreichen, ist folgendes vorgesehen:
- Die Lichtquellen, insbesondere Halbleiter-Laserdioden, sind linear bzw. matrixförmig angeordnet, wobei die Anzahl der Laserdioden sich an der Ausgangsleistung der verwendeten Bauart orientiert. Die Bestrahlung erfolgt in bevorzugter Weise aus drei Richtungen, um einerseits das zu behandelnde Therapieareal räumlich zu bestrahlen und andererseits die geforderte hohe Leistungsdichte zu erreichen,

In Fig. 2 ist ein schematischer Aufbau dargestellt, wobei die Lichtquelle und / oder Lichtquellen 10.1, 10.2 und / oder Lasereinheiten mittels einer Führungseinrichtung und einer Antriebseinheit, insbesondere linear entlang einer Führungsschiene 12, sequentiell über Bestrahlungspositionen 14 (A bis F) in Richtung des Pfeiles 16 bewegt werden. Mit dieser Variante wird keine räumliche Bestrahlung durchgeführt. Die beiden Lichtquellen 10.1 und 10.2 sind auf einem Rahmen oder Schlitten 17 angeordnet, welcher Bestandteil der Führungseinrichtung ist und welcher mittels einer Antriebseinheit 19 entlang der Führungsschiene 12 von einer Bestrahlungsposition zur nächsten bewegbar ist.

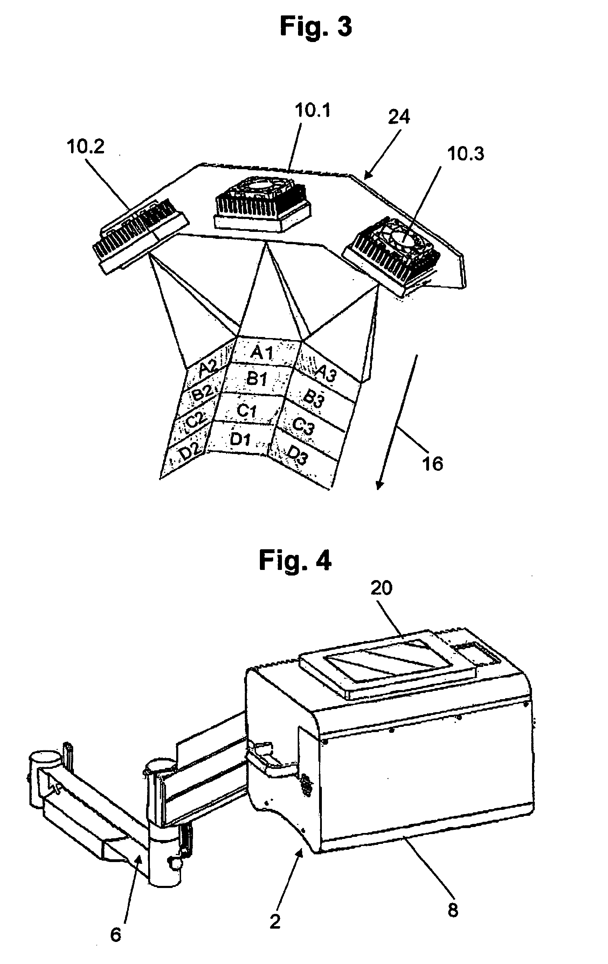

Fig. 3 zeigt schematisch die Anordnung mit drei Lichtquellen 10.1, 10.2, 10.3 und / oder Laserdioden und / oder Lasereinheiten für eine räumliche Bestrahlung, entlang einer mit dem Pfeil 16 angedeuteten Bewegungsrichtung, wobei die verschiedenen Teilflächen A bis D sequentiell bestrahlt werden. Die Lichtquellen 10.1, 10.2, 10.3 sind auf einem Lasersystem 24 angeordnet, welches auf einem hier nicht weiter dargestellten Rahmen samt zugeordneter Führungseinrichtung in Richtung des Pfeiles 16 verschiebbar und zugeordnet zu den verschiedenen Teilflächen A bis D positionierbar ist. Bezüglich einer mittleren Lichtquelle 10.1 sind die beiden seitlichen Lichtquellen 10.2, 10.3 bzw. Laserdioden schwenkbar angeordnet, um eine Anpassung an die Kontur der zu behandelnden Oberfläche in vorteilhafter Weise durchführen zu können. Nachfolgend werden der Einfachheit halber die Lichtquellen als Laserdioden bezeichnet, doch erfolgt hierdurch keine Beschränkung der Erfindung.

Die Vorrichtung ist in bevorzugter Weise in folgende fünf Hauptmodule, deren voran gestellten alphanumerische Bezeichnungen nachfolgend verwendet werden, unterteilt:
- M1 Bestrahlungseinheit 2
- M2 Positioniereinrichtung / Positionierarm / Gelenkarm 6
- M3 Energieversorgungseinheit incl. Sicherheitseinrichtungen 4
- M4 Gerätewagen 8
- (M5 Therapieset).

Das Modul M1 - Bestrahlungseinheit 2- ist in zweckmäßiger Weise in drei Submodule unterteilt:
- M1A Lasereinheit 10
- M1 B Steuerungsmodul
- M1C Gehäuse 18 und Bestrahlungseinheit 2.

Fig. 4 bis 6 zeigen Ansichten einer besonderen Ausführungsform der Bestrahlungseinheit 2 mit einem Gehäuse 18 und einem auf diesem bzw. mit diesem integrierten Display 20. Das Display 20 ist insbesondere Bestandteil einer weiter unten noch zu erläuternden Bedieneinheit. Über ein Kugelgelenk 22 der Positioniereinrichtung ist die Bestrahlungseinheit 2 mit dem Gelenkarm 6 gekoppelt zwecks vorgebbarer und / oder freien Positionierung bzgl. des Therapiebereichs, wobei ein Benutzer zweckmäßig die Handgriffe 23 am Gehäuse ergreift. Das Lasersystem 24 enthält die drei Laserdioden 10.1, 10.2 und 10.3 und ist mittels einer Linearführung 26 verfahrbar und / oder positionierbar. Des Weiteren ist eine Kamera 28 vorgesehen, welche bevorzugt mit der Lasereinheit 24 gekoppelt und positionierbar ist. Der Verfahrweg des Lasersystems 24 ist mittels der Linearführung 26 vorgegeben und beträgt bei dieser Ausführungsform bevorzugt im Wesentlichen 15 cm. Im Rahmen der Erfindung kann die Linearführung 26 auch für eine andere Größe des Verfahrwegs vorgegeben sein. Das Lasersystem 24 enthält die drei Lichtquellen und / oder Lasereinheiten 10.1, 10.2 und 10.3 in räumlicher oder 3D-Anordnung.

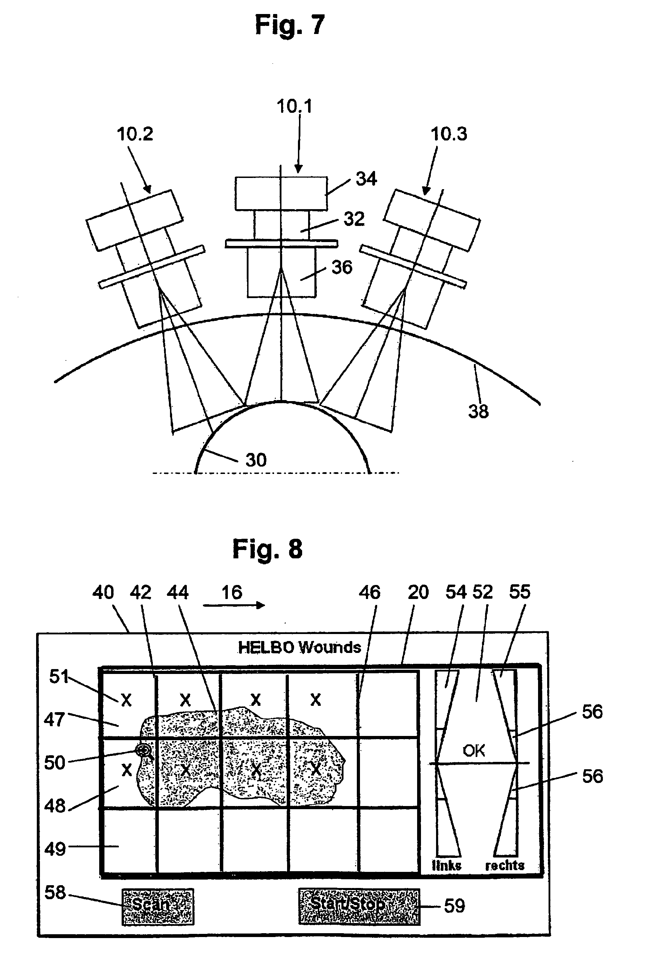

Fig. 7 zeigt die Anordnung der drei Lasereinheiten 10.1 bis 10.3, wobei die seitlichen Lasereinheiten gegenüber der mittleren Lasereinheit 10.1 im Wesentlichen um 20° geschwenkt angeordnet sind und wobei das Drehzentrum sich im Zielgebiet bzw. dem Therapiebereich befindet. Beispielshaft ist mit der Linie 30 die Oberfläche eines mittels der Lasereinheiten bestrahlten dicken Beines angeordnet, dessen Durchmesser 105 mm beträgt. Die drei identischen Lasereinheiten, welche in die Bestrahlungseinheit 2 eingebaut sind, enthalten jeweils eine Laserdiode 32, einen Kühlkörper 34 sowie eine Optik 36. Mittels der Linie 38 ist eine lichtdurchlässige und / oder transparente Schutzscheibe des Bestrahlungsgeräts angedeutet, wobei diese Schutzscheibe insbesondere aus Polycarbonat besteht. Die Lasereinheiten enthalten im Wesentlichen jeweils
- eine Laserdiode mit interner oder externer Kühlung
- eine Kollimationslinse
- zwei Microlens-Arrays zur Strahl-Homogenisierung
- Optik-Fassungen und -Halterungen

Die nachfolgend teilweise als Anforderungen formulierten Merkmale und / oder Funktionen und / oder Eigenschaften der Module und Komponenten sowie deren Zusammenwirken sind einzeln oder in sinnfälliger Kombination in der erfindungsgemäßen Vorrichtung realisiert und / oder werden mit dem erfindungsgemäßen Verfahren verwirklicht.

### M1A Lasereinheit

Die Lasereinheit 10 erfüllt insbesondere folgende Vorgaben:
- Verwendung von Laser als Lichtquelle, wobei das Laserlicht eine Wellenlänge von bevorzugt zumindest näherungsweise 661 nm (+/-20nm) aufweist
- Wellenlänge des Lichtes muss jener entsprechen, die den verwendeten Farbstoff aktiviert
- Leistungsdichte von mindestens 100mW/cm² auf bestrahlter Wundoberfläche für die Aktivierung des Farbstoffes
- Notwendige pro Flächeneinheit auf der Wundoberfläche applizierte Energie bzw. wirksame Dosis: 3-5J/cm²
- Der Abstand der Bestrahlungseinheit wird dem Bestrahlungssystem angepasst, um eine optimale Bestrahlung (korrekte Leistungsdichte etc.) zu gewährleisten. Der Regel-Abstand beträgt 10 cm (Laserdiodenaustrittsstelle bis zur Wundoberfläche), um ein Kamerabild aufnehmen zu können und Bestrahlung mit richtiger Leistungsdichte zu erhalten. Der Mindestabstand beträgt 8cm, um eine Berührung der Wunde durch das Gerät (vor allem bei gekrümmten Oberflächen) zu verhindern. Der Maximalabstand beträgt 12cm, um eine ausreichende Leistungsdichte zu gewährleisten (Messung jeweils im Zentrum des Strahls).
- Das Gehäuse 18 ist derart gestaltet, dass bei gegebenem Abstand der Lasereinheit der Patient nicht berührt wird.
- Mit einer einmaligen Bestrahlung wird eine Wundfläche bestrahlt, die einen Großteil der Wunden abdeckt. Eine Festlegung auf eine definitive Größe ist nicht möglich, da diese Wunden in ihren Formen stark variieren. Ziel ist es aber, eine Wunde, deren Größe in etwa der halben Oberfläche eines menschlichen Unterschenkels entspricht - im Idealfall innerhalb von 10 Minuten - bestrahlen zu können. Innerhalb einer Behandlung (ohne Repositionierung der Bestrahlungseinheit) muss eine ebene Fläche von 15x13cm (bzw. 15x10cm bei Bestrahlung einer gekrümmten Fläche) bestrahlbar sein.
- Diese mindestens zu bestrahlende Wundfläche bezieht sich auf den klinisch wirksamen Bereich in dem die angegebenen 100mW/cm² Leistungsdichte vorhanden sind. Auf die gaußsche Verteilung muss geachtet werden. Im Bedarfsfall sind entsprechende Optiken bzw. Strahl-Homogenisiererzu verwenden. Homogene Bestrahlung der gesamten Fläche, an keinem Bereich sollte die Leistungsdichte von 100mW/cm² unterschritten werden, an Stellen, wo sich die Strahlkegel überlappen, darf die oben angeführte Leistungsdichte überschritten werden.
- Die Bestrahlungsdauer sollte 15 Minuten nicht überschreiten. Behandlungsdauer maximal 15 Minuten, für sehr große Flächen darf diese Dauer überschritten werden, Festlegung jener Region, die innerhalb 15 Minuten behandelbar ist.
- Die Bestrahlung sollte alle drei Dimensionen berücksichtigen. Die Reduktion durch eventuelle Strahlwinkel muss berücksichtigt werden und Maßnahmen sollten gesetzt werden, um Bestrahlungsfehler zu vermeiden (zu geringe Leistungsdichte -> zu wenig Energie). Bevorzugt erfolgt die Bestrahlung aus den drei Raumrichtungen mit einer im Wesentlichen gleichmäßigen Leistungsdichte. Bei Bestrahlung von gekrümmten Oberflächen (z.B. Bein) darf die Leistungsdichte von 100mW/cm² an den Stellen, die weiter von der Lichtquelle entfernt sind, nicht unterschritten werden. An näherliegenden Stellen dürfen folgende Grenzwerte nicht überschritten werden:
   - 500mW/cm² (Grenzwert der Laserklasse 3B)
   - ± 20% der Nennausgangsleistung (gemäß EN 60825)
- Die Konstanz der abgegebenen Lichtleistung ist über die gesamte Bestrahlungsdauer gewährleistet (⇒ Leistungsschwankungen, Ausfall von einzelnen Lichtquellen,.....), wobei insbesondere eine Abstandsregelung und/oder eine konstante Regelung der Ausgangsleistung erfolgen.
- Eine entsprechende Abfuhr der von den Lichtquellen erzeugten Abwärme ist sichergestellt. Durch die erhöhte Anzahl von Lichtquellen, die zur Flächenbestrahlung notwendig sein wird, ist mit erhöhten Temperaturen im Bereich der Lichtquellen zu rechnen. Eine entsprechend ausgelegte Kühlung für die Lichtquellen ist Voraussetzung. Die Kühlung ist auf Dauerbetrieb auszulegen. Auf entsprechende Normen gemäß den maximal zulässigen Oberflächentemperaturen ist zu achten. Die Erwärmung der Lichtquelle bzw. des Gerätes darf die maximale Temperaturwerte der Lichtquelle bzw. der Normvorgaben nicht überschreiten. Erwärmung der Außenflächen: Gemäß Norm 60601-1:1990.: Maximale Oberflächentemperatur für Anwendungsteile 41 °C = Teile, welche mit dem Patienten direkt in Berührung kommen. Maximale Oberflächentemperatur für metallische Teile, welche vom Anwender ständig gehalten werden, beträgt 55°C. Ein unzulässiger Luftzug im Bereich der Wunde (verursacht durch Ventilatoren) wird vermieden.
- Für eine zuverlässige und einfache Messung und Einstellung der abgegebenen Strahlung ist eine Vorrichtung für die Kalibrierung vorgesehen.
- Stoß/schocksichere Aufhängung der Laserdioden, um Schäden durch Anstoßen des Gerätes etc. zu vermeiden. Es erfolgt eine Abfederung von Stößen durch elastische Aufhängung des Laserdiodenträgers oder der gesamten Bestrahlungskopf-Mechanik.
- Die abgegebene Laserleistung ist fix und wird entsprechend der gegebenen Leistungsdichte und dem fix definierten Abstand eingestellt. Es erfolgt eine konstante Regelung der Ausgangsleistung auf einen fix definierten Wert, insbesondere eine Regelungselektronik (Laserdioden-Treiberbaustein).

### Schnittstellen von M1A zu anderen Modulen:

- M1B: Laserdioden werden von M1 B ein- bzw. ausgeschaltet.
- M1B: Befestigung der Laserdioden (samt Kühlung und ev. Optiken) am beweglichen Teil ("Schlitten") der Linearführung. Ein Laserdiodenträger ist als Rahmen ausgebildet, welcher den beweglichen Teil der Linearführung bildet.
- M1B: Einbau der Laserdioden-Treiberbausteine
- M1C: Lüftungsauslässe im Gehäuse
- M2/M3: Energieversorgung der Dioden

### M1 B Steuerungsmodul:

Das Steuerungsmodul umfasst:
- Einen Linearantrieb zur linearen Bewegung und Positionierung der Lasereinheit (z.B. mit Schrittmotor und Inkrementalgeber zur Positionsbestimmung). Die lineare Beweglichkeit der Lasereinheit ist entlang der Hauptausdehnungsrichtung der Bestrahlungseinheit vorgegeben. Die Länge der Lineareinheit ergibt sich aus der Länge der maximal bestrahlbaren Fläche, bevorzugt im Bereich von 10 bis 25 cm, insbesondere im Bereich von 14 bis 20 cm.
- Je eine Antriebseinheit zur Positionierung der seitlichen, schwenkbaren Laserdioden (z.B. mit Schrittmotor und Inkrementalgeber zur Positionsbestimmung. Zur zentralen Laserdiode angeordnet, um sowohl eine gewölbte (z.B. Bein) als auch ebene Flächen bestrahlen zu können (z.B. Rücken). Die seitlich angebrachten Dioden sind in einem bevorzugt vorgebenen Winkelbereich von 10° bis 30°, insbesondere von im Wesentlichen 20° schwenkbar.
- Wenigstens eine Kamera, die ein Bild der bestrahlbaren Fläche liefert. Bevorzugt ist eine Farbkamera vorgesehen, die ein Bild der gesamten bestrahlbaren Fläche aufnimmt und liefert. Falls im gegebenen Abstand zur Wundoberfläche der Aufnahmewinkel der Kamera nicht ausreicht, können optional mehrere Kameras oder eine fahrbare Kamera (eigene Linearführung oder Bewegung gemeinsam mit der Lasereinheit), die die Fläche abscannt, verwendet werden. Zu beachten ist, dass bei einer gekrümmten Wundoberfläche der Aufnahmewinkel des Bildes nicht mehr der Strahlrichtung der äußeren Laserdioden entspricht. Ferner ist bevorzugt wenigstens eine zusätzliche Lichtquelle vorgesehen, um ein gutes Kamerabild zu erhalten.

### Abstandssensoren:

- Ein Abstandssensor im Bereich der zentralen Laserdiode, welcher sich mit dieser Diode mit bewegt.
- Einer an einem (optional beiden) langen Ende der Bestrahlungseinheit, welcher die Distanz der Bestrahlungseinheit/Laserdioden zur Wunde misst.
- Je ein Abstandssensor, welcher die Entfernung von den äußeren, schwenkbaren Dioden zur Wundoberfläche misst. Die Abstandssensoren sind für zentrale und seitliche Laserdioden sowie fix an einem Ende der Bestrahlungseinheit vorgesehen. Eine in bevorzugter Weise ausfallsichere Variante enthält zwei Abstandssensoren auf zumindest der zentralen Laserdiode. Der oder die Abstandssensoren sind insbesondere als Ultraschall- oder Lasersensoren ausgeführt. Die Genauigkeit der Messung liegt bevorzugt im Bereich von ±5mm.

### Display insbesondere mit Touchscreen:

- Die Eingabe erfolgt mit Stift oder Finger, wobei die Bedienung mit Einweghandschuhen möglich sein soll. Die Ausrichtung der Anzeigen ist derart vorgegeben, dass ein auf der Seite des Gerätewagens stehender Anwender jene leicht und/oder gut erkennen bzw. interpretieren kann. Das Display (mindestens 8 Zoll) dient zur Darstellung des Kamerabildes, Überblendung mit einem Raster, Darstellung der ausgewählten Felder, Darstellung von rot/grünen Balken zur Unterstützung der Abstands-Positionierung. Das Display ist derart angeordnet, dass auch Anwender mit geringer Körpergröße das Display in jeder Position ablesen können.

### Eingabetasten:

### - Scan (Kamera-Aufnahme starten)

Start/Stop (Bestrahlung starten/stoppen). Bevorzugt ist eine Folientastatur mit allen benötigten Eingabetasten vorgesehen. Ferner sind die Eingabetasten optional in das Display integriert. Weiterhin sind die Eingabetasten derart ausgebildet und/oder angeordnet, dass durch einen Tastendruck die eingerichtete Position der Bestrahlungseinheit nicht verändert wird.

### Not-Aus-Taster:

### - Not-Aus-Taster an Bestrahlungseinheit und Gerätewagen, bei deren Betätigung alle Antriebesmotoren und die Laserstrahlung gestoppt werden.

### Steuerungssoftware:

Die Steuerungssoftware, beispielsweise in Form eines Embedded Systems, erfüllt und / oder steuert die nachfolgend beschriebenen Funktionen:
- Prozessor und elektronische Komponenten:
- Prozessor, auf dem die Steuerungssoftware läuft und Elektronikpaket zur Steuerung von Antrieben, Sensoren, Kamera, Display, Eingabetasten und Not-Aus. Vorgesehen ist ferner die Absicherung der Funktionen der Steuerungssoftware/Elektronik, um bei Ausfall des Systems die Not-Aus-Funktion zu aktivieren (Watchdog Hardware / Software).
- Eingriffschutz an der Unterseite zum Schutz von Personen beim "Hineingreifen ins Gerät: Gelangt ein Objekt innerhalb eines definierbaren Abstands zur Laserstrahl-Austrittsöffnung, so wird der Pause-Modus aktiviert (Laser aus).
- Mechanische Komponenten: Rahmen, welcher alle Bauteile, insbesondere Linearführung, Antriebe, Kamera, Display, Bedienelemente, Sensoren, Zugangsschutz für Dioden, trägt.
- Laserbetriebsanzeige gemäß einschlägiger Normen, wie z.B.:
   Grün: Laser bereit (Schlüsselschalter betätigt. Gerät eingeschaltet, und Temperatur der Laserdiode liegt im korrekten Bereich.
   Gelb / Orange: für Laseremission - Start wurde gedrückt - Gerät gibt Lichtstrahlung ab Rot: Fehler (nicht zwingend erforderlich)
   Die Anzeigen sind gemäß einschlägiger Normen (EN60825, EN60601-1-22) entweder im Display integriert oder als eigenständige Leuchte (z.B. LED, LED-Streifen, etc.) ausgebildet.
- Manuelle Wiedereinschaltrichtung:
   Die Bestrahlung darf nach Stromausfall o. ä. nicht automatisch fortgesetzt werden
- Speicherung der Betriebszeiten:
   Es erfolgt eine dauerhafte Speicherung der Gesamtbetriebszeit des Gerätes sowie der Laserbetriebszeit der einzelnen Dioden.

### Schnittstellen von M1B zu anderen Modulen:

- M1A: ein/ausschalten der Laserdioden und diverse Überwachungsfunktionen durch direkte Ansteuerung/Abfrage der Laserdioden-Regelung (spannungsgeregelt); Ansteuerung / Überwachung für alle drei Laserdioden.
- Dioden ein / aus, spannungsgeregelt. Überwachung der Laserdioden-Temperatur durch Spannungssignal von der Laserdioden-Regelung. Freigabe der Bestrahlung erst, wenn die Temperatur der LD im festgelegten Bereich liegt. Überwachung des Laserdiodensystems, Warnung falls Abweichung vom erlaubten Bereich (Hinweis auf Beschädigung). Überwachung der Drehzahl der Laserdioden-Lüfterkühler (Alarm, wenn Kühler stillsteht). Optional: Regelung der Laserleistung durch Spannungssignal.
- M1A: Befestigung der (drei) Laserdioden (samt Kühlung und ev. Optik) am beweglichen Teil ("Schlitten") der Linearführung. Rahmen, welcher die Dioden aufnimmt als Teil der Linearführung. Schnittstelle zur Laserdiodeneinheit sind insbesondere Gummibacken / Gummiringe, o. ä.
- M1A: Einbau der Laserdioden-Treiberbausteine:
   Platz zum Einbau der (drei) Laserdioden-Treiberbausteine incl. Kühlkörper/Lüfter ist innerhalb der Bestrahlungseinheit vorgesehen (Maße ca. 120x70x60 mm; Einbau stationär oder am "Laserschlitten").
- M1C: Verbindung des Rahmens mit dem Gehäuse, sowie Einbau des Displays, der Eingabetasten / Folientastatur, Not-Aus ins Gehäuse. Im Bereich der Verbindung Gelenkarm - Bestrahlungseinheit ist bevorzugt eine Rotationsbewegung, insbesondere um eine horizontale Achse entlang der Bestrahlungseinheits-Querachse ermöglicht, wobei die Bestrahlungseinheit in der gewählten Position bevorzugt automatisch arretierbar ist.
- M2 / M3: Energieversorgung
- M2: Befestigung der Bestrahlungseinheit an Positioniereinrichtung / am Positionierarm.

### M1C Gehäuse Bestrahlungseinheit:

- Gehäuse mit eingepassten Bedienelementen der Benutzeroberfläche und mit Lüftungsauslässen: seitliche (oder oben gelegene, aber möglichst gegen direkten Flüssigkeitseintritt geschützte) Luftein- und auslässe, eventuell mit eigenem Lüfter, um einen konstanten leichten Luftzug zu ermöglichen, der die Abwärme der Laserdioden nach außen transportiert. Einbau der Bedienelemente bevorzugt an der Oberseite des Gehäuses. Die Unterseite des Gehäuses ist grundsätzlich offen, da dort die Laserstrahlung austritt. Optional: Eigener Touchscreen (unabhängig von der Bestrahlungseinheit) mit eigener Befestigung (Gelenksarm) am Gerätewagen.
- Die Geräteunterseite sollte variabel verschließbar sein, d.h. nur die jeweilige Laseraustrittsstelle ist offen (bzw. durch eine Glas-/Kunststoffplatte abgedeckt), die restliche Öffnung ist durch eine Art "Vorhang" oder ähnliches verschlossen. Starre Abdeckung der Unterseite der Bestrahlungseinheit als Eingriffs- und Staub-/Schmutz-Schutz: durchsichtige Polycarbonat (PC-)Platte, gebogen (r=240mm) entlang der Gerätelängsachse, 2mm stark.
- Griffe zur manuellen Positionierung der Bestrahlungseinheit über dem Wundareal. Mindestens zwei seitlich am Gehäuse angebrachte Griffe.
- Beachtung des Gehäuseableitstroms:
   AKM1 C4: Gehäuseableitstrom ist innerhalb Grenzwerten (z.B. durch Versorgung der Bestrahlungseinheit mit Kleinspannung und Anwendung aller relevanten Bereiche der EN60601).
- Gehäuseform sollte an die Körperform angepasst sein, damit die Wundoberfläche nicht berührt wird. Das Gehäuse muss derart gestaltet sein, dass bei gegebenem Abstand der Lasereinheit der Patient nicht berührt wird, vor allem bei gekrümmten Behandlungsarealen.
- EMV Schirmung der Elektronik durch Gehäuse (Material)
- Schutz gegen Feuchtigkeit von außen:
   Das Gehäuse sollte gegen Flüssigkeitseintritt (vor allem von oben) in die darunterliegende Elektronik schützen (Dichtungen bei den eingepassten Bedienelementen, etc.).
- Verwendung geeigneter Materialien für das Gehäuse, insbesondere eloxiertes oder pulverbeschichtetes Aluminium.

### M2 Modul Positioniereinrichtung:

- Ausführung insbesondere mit Gelenkarm 6 und / oder Gelenkstativ, welche idealerweise mit einer zentralen Schnellfixierung ausgestattet sind. Gelenkarm / Gelenkstativ mit zentraler Schnellfixierung, 100%ig fixe Positionierung während gesamter Behandlungsdauer; Arretierung in Transportposition. Die Positioniereinrichtung enthält bevorzugt zur Verbindung mit der Bestrahlungseinheit 2 das bereits erwähnte Kugelgelenk 22, wobei auch dieses zweckmäßig mit zentraler Schnellfixierung und / oder festen Positionierung ausgerüstet ist.
- Die Bestrahlungseinheit sollte jede Körperstelle bestrahlen können (bei liegendem Patient, Anwendung im Bereich der Höhe der gängigen Patientenbetten). Reichweite horizontal von der Außenkante des Gerätewagen bis zum Mittelpunkt der Bestrahlungseinheit: min. 100cm. Reichweite vertikal (Strahlausgangsebene von 63 bis 136 cm Höhe).
- Kabel für Spannungsversorgung von der Energieversorgungseinheit zur Bestrahlungseinheit. Kabel sollte wenn möglich verdeckt im bzw. entlang des Gelenkarms verlegt werden.

### M3 Modul Energieversorgungseinheit incl. Rechner und Sicherheitseinrichtungen:

- Gehäuse mit eingebautem zentralen Ein-/Ausschalter.
- Spannungsversorgung mit Netzanschlussleitung. Transformation der Netzspannung in Kleinspannung, welche über Kabel an die Bestrahlungseinheit weitergeleitet wird.
- EMV Verträglichkeit der Spannungsversorgung bzw. Abschirmung durch das Gehäuse unter Einhaltung der EMV-Vorschriften.
- Netzgerät oder Transformator:
   Spannung 5V (Laserdioden, Echtzeitsystem), und 12V (PC-System / Rechner) Leistungsaufnahme Laserdioden ca. 6A
- Rechner, welcher zweckmäßig als ein PC-System ausgebildet ist und bedarfsweise separat angeordnet und über ein Kabel verbunden ist.

### M5 Modul Therapieset:

Der als Therapieset bezeichnete Bestandteil besteht aus den für jede einzelne aPDT-Anwendung notwendigen Materialien:
- Photosensitizer: Aufgrund der Verfügbarkeit von HELBO Blue Kutan, welches für die Anwendung an Hautoberflächen fertig entwickelt und bereits zugelassen ist, wird diese Farbstofflösung für die Anwendung mit diesem Gerät in Verwendung kommen. So sind folgende Parameter als gegeben zu sehen - Wellenlänge nahe der 661 nm, des Absorptionsmaximums vom photoaktiven Bestandteil.
- Die Photosensitizer-Füllmenge pro Therapieset sollte ausreichen, um die maximal bestrahlbare Wundfläche anfärben zu können. Mit 0,5 ml HELBO Blue Kutan können ca. 50 cm² angefärbt werden.
- Optional / zukünftige Varianten:
   Therapiesets mit unterschiedlich großen Füllmengen für verschiedene Wundgrößen.
- Der weitere Inhalt des Therapiesets sollte alle Hilfsmittel enthalten, die für die Applikation, Tupfen, Spülen, etc. der Wunde notwendig sind.
- Alle Einzelteile des Therapiesets sind steril in einer geeigneten Verpackung in Verkehr zu bringen.

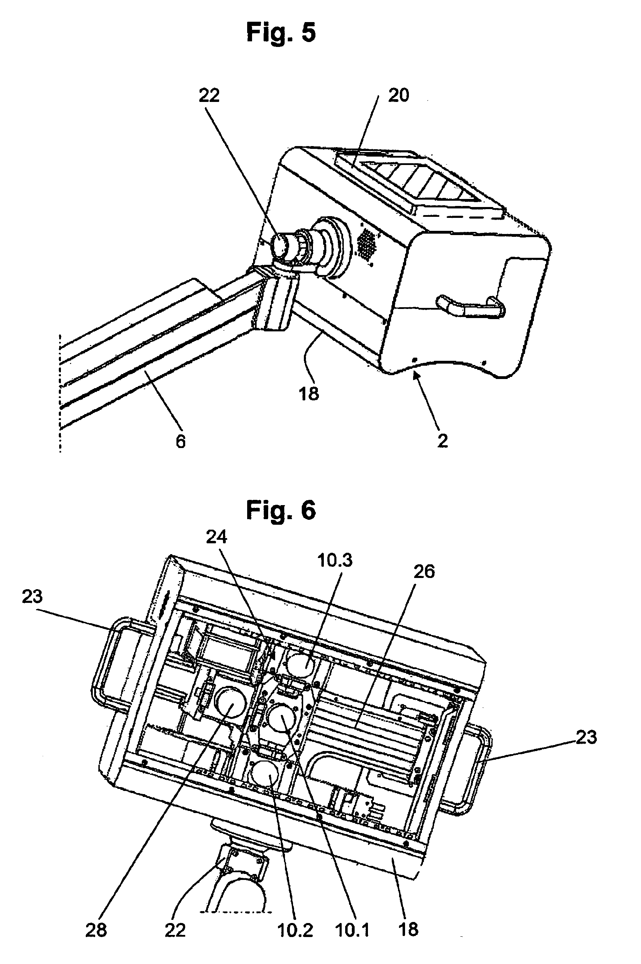

Fig. 8 zeigt eine Ansicht der Bedieneinheit 40, welche bevorzugt auf dem Gehäuse der Bestrahlungseinheit angeordnet ist. Die Bedieneinheit 40 enthält das Display 20, auf welchem das mit der Kamera erfasste und mit dem Rechner verarbeitete Live-Kamera-Bild 42 des Wundareals eingeblendet und die Wundfläche 44 dargestellt wird. Ferner wird ein insbesondere mittels Rechner erzeugtes Rasterfeld 46 eingeblendet und dem Live-Kamera-Bild 42 überlagert. Jedes der bevorzugt quadratischen Rasterfelder entspricht einem mittels der Lasereinheit erzeugten Bestrahlungsfeld. Hierbei ist das anhand von Fig. 6 erläuterte Lasersystem mit drei Laserdioden zugrunde gelegt, mittels welchem gleichzeitig jeweils drei Bestrahlungsfelder 47, 48, 49 erzeugbar sind. Die Bestrahlungsfelder 47, 48, 49 liegen nebeneinander und quer zu der mittels des Pfeiles 16 angedeuteten Bewegungsrichtung des Lasersystems. Aufgrund der Führungseinrichtung und der Antriebseinheit wird das Lasersystem sequentiell in der Bewegungsrichtung 16 verfahren und derart erfindungsgemäß positioniert, dass nacheinander die in der Bewegungsrichtung 16 nebeneinander liegenden Bestrahlungsfelder derart bestrahlt werden können, dass eine matrixartige Bestrahlung entsprechend dem gesamten Rasterfeld 46 durchgeführt wird. Damit nur diejenigen die Wundfläche 44 erfassenden Rasterfelder bestrahlt werden, erfolgt mittels geeigneter Markierungsmittel deren Markierung, welche hier durch Kreuze X angedeutet wird. Ist das Display 20 als Touch Screen ausgebildet, so erfolgt die Markierung durch Antippen oder Berühren der genannten Felder. Alternativ kann die Markierung beispielsweise mittels des Rechners oder des PC-Systems durch einen Mausklick 50 des jeweiligen Feldes erfolgen. Entsprechend der erfolgten Auswahl werden bei Durchführung der Bestrahlung nur die entsprechend markierten Felder des Wundareals bzw. der Wundfläche 44 bestrahlt.

Das Display 20 umfasst ferner eine Abstandsanzeige 52, mit welcher die Positionierung der Bestrahlungseinheit zum Wundareal 44 angezeigt wird. Sind die Balken oder dreieckförmigen Symbole 54, 55 beispielsweise insgesamt rot, so ist die Bestrahlungseinheit an beiden Enden zu weit entfernt. Sind hingegen die Balken oder Symbole 54, 55 zumindest im Bereich der Spitzen 56, sowohl links und rechts, beispielsweise grün, so ist die Bestrahlungseinheit auf beiden Seiten im richtigen Abstand positioniert. Ferner enthält die Bedieneinheit 14 eine Scanntaste 58 und eine Start- /Stopptaste 59.

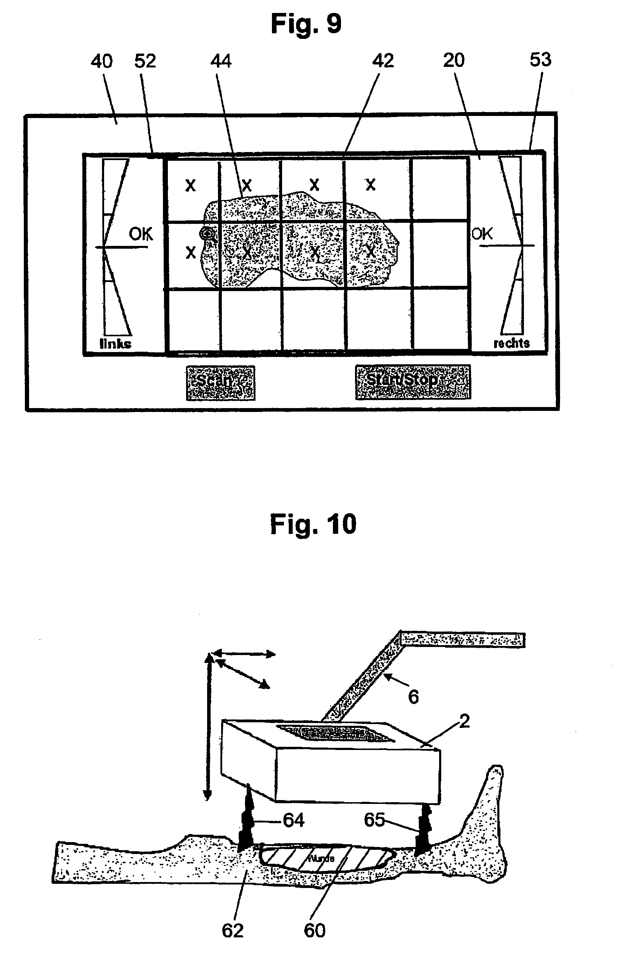

Fig. 9 zeigt eine alternative Ausgestaltung der Bedieneinheit 40 bzw. der Benutzeroberfläche des Displays 20, wobei die Abstandsanzeige in zwei Teile 52, 53 unterteilt ist, welche links bzw. rechts neben dem Kamerabild 42 angeordnet sind. Hierdurch ist eine verbesserte intuitive Anordnung entsprechend der räumlichen bzw. geometrischen Zuordnung der Bedieneinheit zum Wundareal erreicht.

Nachfolgend werden die Funktionsweise der Vorrichtung sowie die verschiedenen Verfahrensschritte näher erläutert:

### Schritt 1: Positionierung der Bestrahlungseinheit über der Wunde

A Bestrahlung von "langen Wunden", wobei die Länge der Wunde in Richtung der Längsachse der Bestrahlungseinheit und / oder der Bewegungsrichtung größer ist als zwei Bestrahlflächen: Die Bestrahlungseinheit 20 wird vom Behandler mithilfe der erwähnten Handgriffe parallel über der Wundfläche positioniert. Die beiden Abstandssensoren sind jeweils an einem Ende der Bestrahlungseinheit 2 positioniert. Der eine Abstandssensor ist auf dem Lasersystem bevorzugt an der zentralen Diode angeordnet, welche initial an einem Ende der Bestrahlungseinheit 2 positioniert wird. Der zweite Abstandssensor ist fest am anderen Ende der Bestrahlungseinheit 2 angeordnet. Die beiden Abstandssensoren messen den jeweiligen Abstand von der Bewegungsebene der zentralen Laserdiode zur darunter liegenden Wund-Oberfläche bzw. dem Wundareal.
B Bestrahlung von kurzen Wunden, deren Länge in Richtung der Längsachse maximal zwei auswählbare Bestrahlflächen groß ist. Im Gegensatz zur Variante A wird der Abstand beim Einrichten nur an einem Ende der Bestrahlungseinheit 2 gemessen, bevorzugt mit dem Abstandssensor bei der zentralen Laserdiode. Wenn dieser an einem Ende korrekt ist, kann die Wunde gescannt werden. Im Folgenden ist dann die Auswahl einer Bestrahlungsfläche nur möglich, sofern der Abstand korrekt ist. Es sei festgehalten, dass beim Scannen sowohl das Kamerabild als auch der Abstand ermittelt wird.

Fig. 10 zeigt die Positionierung der Bestrahlungseinheit 2 über eine Wunde 60 an einem Unterschenkel 62. Die an der Positioniereinrichtung 6 befestigte bzw. angelenkte Bestrahlungseinheit 2 ist entsprechend der vorgenannten Variante A vom Behandler parallel über der Wundfläche 60 positioniert, wobei die Bestrahlungseinheit im Wesentlichen parallel zur Längsachse der Wunde liegt. Mit den Pfeilen 64, 65 ist die Messstrahlung der Abstandssensoren angedeutet. Mittels der anhand von Fig. 8 oder Fig. 9 erläuterten Abstandsanzeigen erfolgt die grafische, insbesondere farbliche Darstellung des Abstands im Display, jeweils mit einem Symbol oder Balken für jeden der beiden Abstandssensoren. Liegt der Abstand im richtigen vorgegebenen Bereich, so leuchten beispielsweise grüne Balken auf, ansonsten rote. Es bedeutet beispielsweise:
- Dunkelgrün: Der Abstand vom vorgegebenen Idealabstand, beispielsweise 100mm beträgt +/- 5mn und ist im Wesentlichen eingehalten.
- Hellgrün: Der Abstand vom Idealabstand beträgt +/- 10mm; kleine Toleranz.
- Hellrot: Der Abstand vom Idealabstand beträgt +/- 20mm; vergrößerte Toleranz.
- Dunkelrot: Der Abstand vom Idealabstand ist größer als 20mm; zu großer bzw. unzulässiger Abstand.

Die Freigabe zur Bestrahlung, und zwar durch Aktivierung der Start-/Stopptaste darf nur dann erfolgen, wenn der richtige Abstand, insbesondere der durch grün dargestellte Idealabstand eingestellt ist und zudem die Betriebstemperatur der Laserdioden im definierten Bereich liegt.

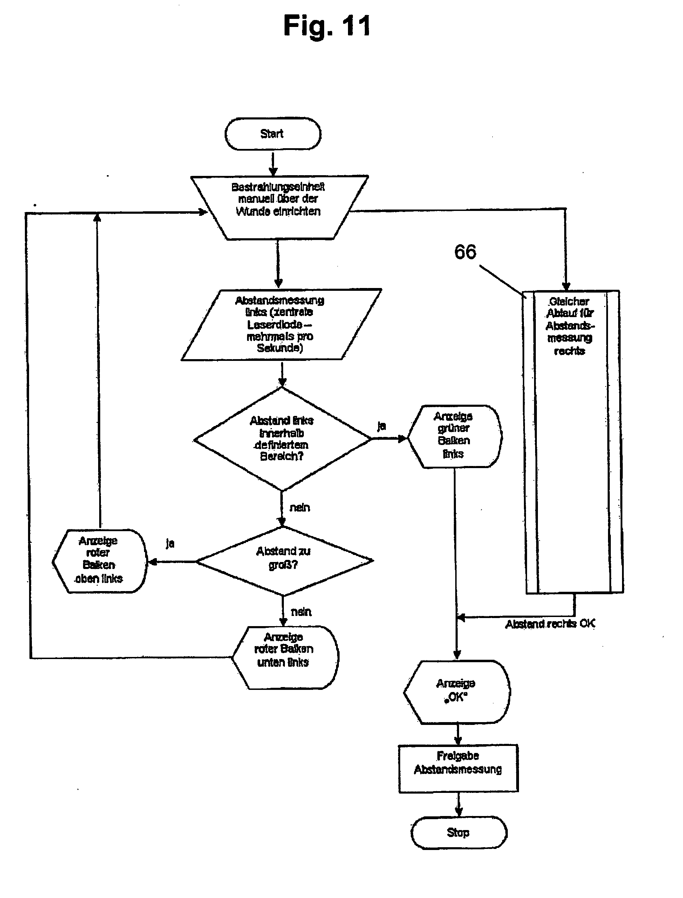

Fig. 11 zeigt das diesbezügliche Flussdiagramm zur Positionierung der Bestrahlungseinheit. Für den linken Abstandssensor ist das Flussdiagramm komplett ausgeführt, während die analoge Abstandsmessung mittels des rechten Abstandssensors durch den Block 66 angedeutet ist.

### Schritt 2: Ausrichtung der äußeren schwenkbaren Laserdioden

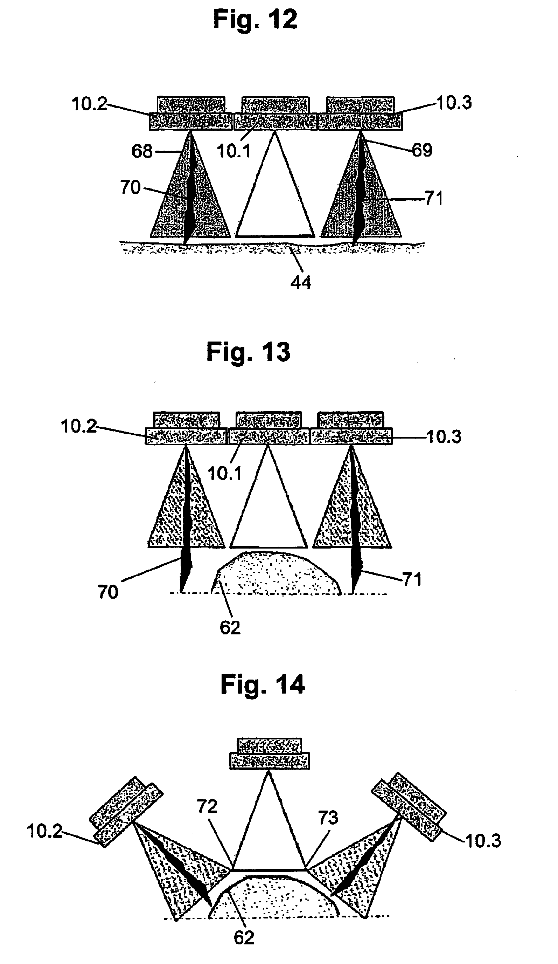

Dieser Verfahrensschritt wird für die insbesondere anhand von Fig. 3, 6 und 7 erläuterte Ausführungsform der Vorrichtung durchgeführt und anhand der Fig. 12, 13 und 14 näher erläutert. Der Abstand der mittleren Laserdiode 10.1 ist bereits gemäß Schritt 1 korrekt eingerichtet. Jeweils ein Abstandssensor 68,69 an den äußeren Dioden 10.2 bzw. 10.3 misst den Abstand zur Wund-Oberfläche. Gemäß Fig. 12 erfolgt auf eine im Wesentlichen ebene Wundfläche 44 eine ebene Bestrahlung, wobei eine Anpassung der beiden äußeren Dioden 10.2 bzw. 10.3 nicht erforderlich ist. Mittels Pfeilen 70, 71 ist die Messstrahlung der Abstandssensoren angedeutet, während die Laserstrahlung jeweils dreieckförmig angedeutet ist. Fig. 13 und Fig. 14 zeigen eine räumliche Bestrahlung eines Unterschenkels 62. Die Verfahrrichtung der Laserdioden 10.1, 10.2 und 10.3 erfolgt senkrecht zur Blattebene nach hinten bzw. in das Bild hinein. Fig. 13 zeigt die Ausgangsposition, wobei der Abstand der mittleren Diode 10.1 bereits ausgerichtet ist. Die beiden seitlichen Dioden 10.2, 10.3 müssen noch an die Beinkrümmung angepasst werden, da die Abstandssensoren zunächst einen zu großen Wert liefern. Wie in Fig. 14 dargestellt, werden die beiden äußeren bzw. seitlichen Dioden mittels zugeordneter Antriebe automatisch derart nach innen geschwenkt, bis der richtige Abstand erreicht ist. Das Drehzentrum ist jeweils bevorzugt der Berührungspunkt 72, 73 der als Dreiecke dargestellten Strahlkegel. Es ist von besonderer Bedeutung, dass die Ausrichtung und / oder Abstandsmessung der seitlichen Dioden 10.2, 10.2 kontinuierlich erfolgt, und zwar bevorzugt mehrmals pro Sekunde. Hierdurch wird in bevorzugter Weise auf Patientenbewegungen reagiert und / oder beim Verfahren der Lasereinheit der Abstand an sich ändernde anatomische Gegebenheiten angepasst.

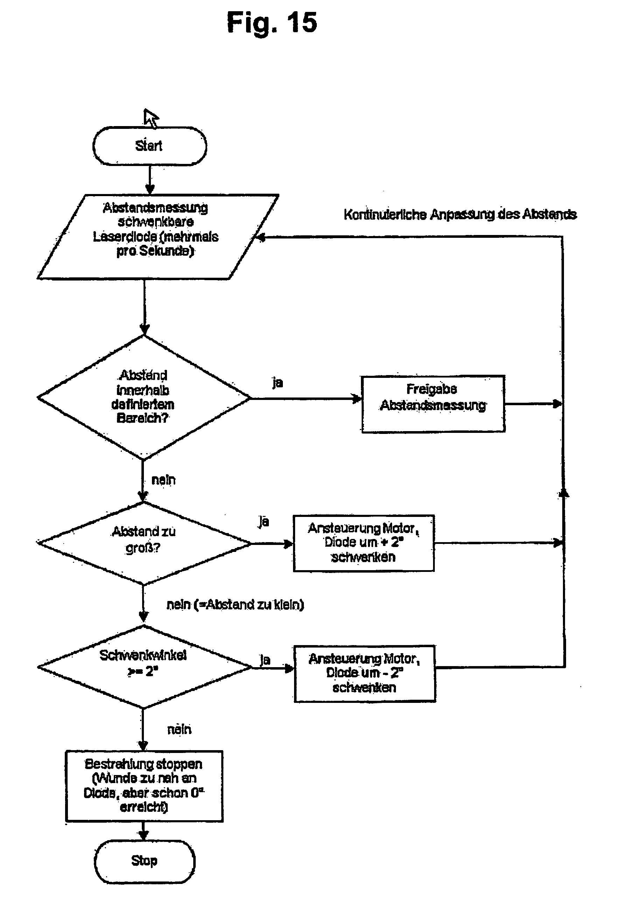

Fig. 15 zeigt das Flussdiagramm zur Positionierung jeweils der beiden schwenkbaren seitlichen Dioden.

### Schritt 3: Abbildung der Wundfläche

Nach Positionierung der Bestrahlungseinheit nimmt die in der Bestrahlungseinheit integrierte Kamera ein Bild der Wunde auf, welches in der Größe zumindest näherungsweise und / oder genau jener Fläche entspricht, welche bestrahlt werden kann. Alternativ kann das Kamerabild aus mehreren Einzelbildern zusammengesetzt werden, wobei bevorzugt mehrere Kameras oder eine fahrbare Kamera vorgesehen ist. Wie bereits anhand von Fig. 8 erläutert, wird das mit der oder dem Kameras aufgenommene Bild der maximal bestrahlbaren Wundfläche im Display dargestellt und mit einem Raster überblendet, welches verschiedene Bestrahlungspositionen darstellt und / oder diesen entspricht.

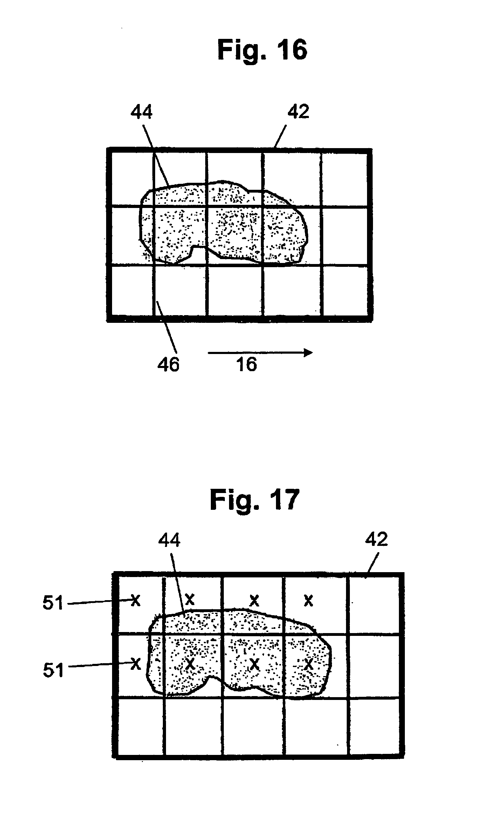

Fig. 16 zeigt das Kamerabild 42 mit der Wundfläche 44 und das eingeblendete Raster 46 im Display. Das Raster in Richtung quer zur Bewegungsrichtung 16 ist in drei Abschnitte unterteilt, wobei jedes Rasterfeld in dieser Richtung der jeweiligen Bestrahlungsposition der drei Laserdioden entspricht. Entlang der Bewegungsrichtung 16 ist beispielsweise eine Unterteilung in fünf Positionen vorgegeben.

### Schritt 4: Auswahl der Bestrahlungsfläche

Auf der Basis des gemäß Schritt 3 werden jene Rasterfelder definiert, welche bestrahlt werden sollen, wie in Fig. 17 dargestellt. Initial ist zunächst noch kein Feld ausgewählt und durch Auswahl des jeweiligen Feldes wird dieses selektiert. Bei Ausführung des Displays als Touch Screen oder Touch Panel ändert ein Fingerdruck auf das entsprechende Rasterfeld die Auswahl des Feldes. In bevorzugter Weise werden ausgewählte Felder markiert und / oder optisch abweichend dargestellt, beispielsweise schattiert oder schraffiert, wobei das aufgenommene Original-Kamerabild noch erkennbar sein muss. Beispielshaft sind entsprechend Fig. 8 die Markierungen 51 durch Kreuze hier dargestellt.

### Schritt 5: Start der Bestrahlung.

Durch Drücken der in Fig. 8 dargestellten Start-/Stopptaste 59 wird die Bestrahlung gestartet. Es ist von besonderer Bedeutung, dass die Start-/Stopptaste nur dann aktiv ist, wenn der Abstand richtig gewählt ist, wobei die Freigabe gemäß der Abstandsmessung erfolgt und zudem mindestens ein Feld ausgewählt ist. Die Lasereinheit beginnt von einem Startpunkt, welcher an einem der beiden Endpunkte der Führungseinrichtung insbesondere der Linearführung ist, die Bestrahlung. Jede Position entsprechend der Positionierung der Lasereinheit in der Bewegungsrichtung bzw. Verfahrrichtung der Linearführung wird für eine definierte Zeit bestrahlt, und zwar je nach erreichter Leistungsdichte in Abhängigkeit von der Ausbildung der Lasereinheit. Nachfolgend wird die Lasereinheit bzw. das Lasersystem zur nächsten Position weiter bewegt, in welcher die Bestrahlung fortgesetzt wird. Es sei darauf hingewiesen, dass ein Auswahlfeld nicht unbedingt einer Bestrahlposition entsprechen muss. Die Größe des von einer Laserdiode auf einmal bestrahlten Gebietes oder Flächenbereiches in Richtung der Verfahrrichtung kann ggf. kleiner sein als die Ausnehmung des oder der Rasterfelder. In einem solchen Fall sind zur Bestrahlung eines ausgewählten Feldes zwei oder mehr Bestrahlungspositionen notwendig. Beträgt beispielsweise die Bestrahldauer pro Feld 40 Sekunden, so entspricht ein ausgewähltes Feld fünf Bestrahlpositionen von beispielsweise 6 mm. Erfindungsgemäß werden nur jene Felder bestrahlt, welche ausgewählt worden sind. Die Diode für eine nicht ausgewählte Position wird an der jeweiligen Position abgeschaltet. Ist an einer Position in der Verfahrrichtung kein Feld bzw. keines der drei nebeneinander mehr zur Fahrrichtung vorhandenen Felder ausgewählt, so wird die Lasereinheit sofort zur nächsten Positionen weiter verfahren.

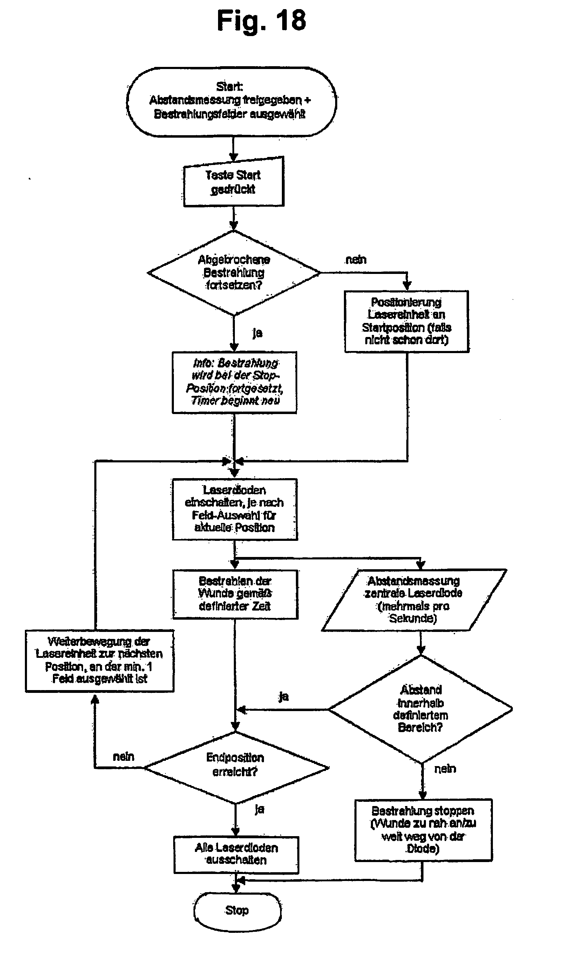

Fig. 18 zeigt das Flussdiagramm der Bestrahlung, und zwar incl. der kontinuierlichen Abstandskontrolle. Parallel bzw. gleichzeitig hierzu wird die Abstandsmessung und Anpassung der seitlichen Laserdioden durchgeführt, wie in Fig. 15 dargestellt.

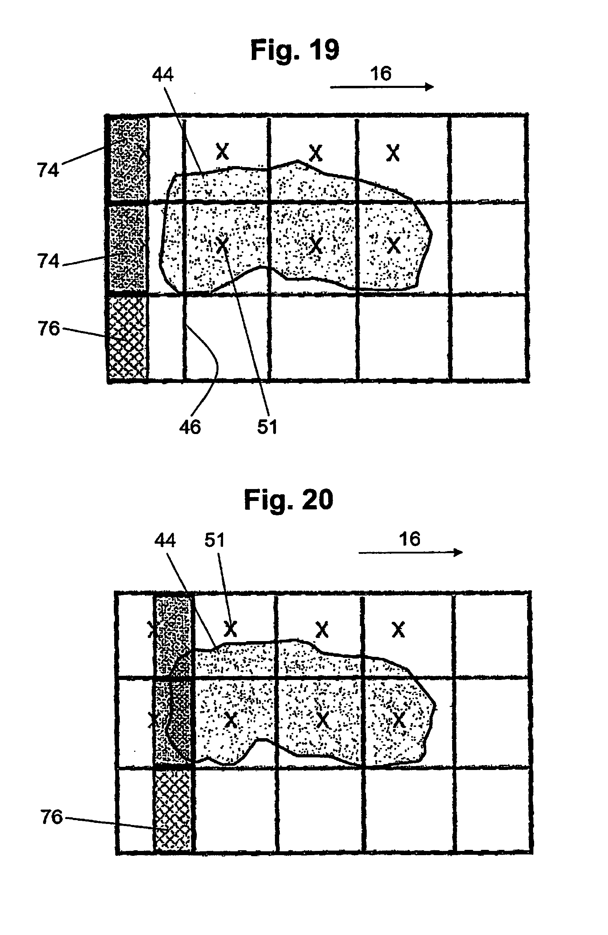

Fig. 19 und 20 zeigen Kamerabilder der Wunde bzw. Wundfläche 44 mit dem Raster 46 und die entsprechend den Markierungen 51 ausgewählten Felder. Die grau unterlegten Flächen 74 stetlen die darunter liegenden, zum jeweiligen Zeitpunkt durch die Laserdioden physisch bestrahlten Flächen dar, wobei die zugeordneten Laserdioden eingeschaltet sind. Hingegen werden die mit gekreuzter Schraffur unterlegten Flächen 76 nicht bestrahlt, da die zugeordnete Laserdiode ausgeschaltet ist. Fig. 19 zeigt eine erste Bestrahlungsposition des Lasersystems und Fig. 20 zeigt eine zweite Bestrahlungsposition des Lasersystems in der Bewegungsrichtung 16, und zwar jeweils in den gleichen Rasterfeldern 46.

### Schritt 6: Kontinuierliche Abstandskontrolle

Um Abstandsänderungen durch Bewegung des Patienten oder ähnliches während der Therapie zu vermeiden, wird erfindungsgemäß die Distanz zwischen der zentralen Laserdiode und der Wundoberfläche laufend kontrolliert. Während der Bestrahlung des Zielgebietes und / oderwährend der linearen Weiterbewegung der Lasereinheit wird in bevorzugter Weise der Abstand der zentralen Laserdiode kontinuierlich überwacht. Liegt der Abstand außerhalb des definierten Bereiches, wird die Behandlung bzw. Bestrahlung unterbrochen. Sobald der geforderte Abstand wieder hergestellt ist, kann nach einer Unterbrechung die Therapie fortgesetzt werden, insbesondere durch drücken der Start-/Stopptaste, und zwar ausgehend von der aktuellen Position.

### Schritt 7: Therapieende

Die Bestrahlung wird beendet, wenn
a) alle Felder bestrahlt wurden;
b) die Therapie automatisch aufgrund eines falschen Abstandes unterbrochen und nicht wieder fortgesetzt wurde;
c) eine maximale Gesamt-Therapiezeit wird. Damit wird erreicht, dass die Lichtquellen im Fehlerfall automatisch abgeschaltet werden;
d) die der Taste Start/Stopp nach dem Therapiestart und vor einem Therapieende gedrückt wird;
e) der Not-Aus-Taster gedrückt wird.

Eine automatische Abschaltung der Lasereinheit der Linearbewegung wird durchgeführt, nachdem alle ausgewählten Felder bestrahlt wurden, wobei bevorzugt ein akustischer und / oder optischer Feedback gegeben wird. Um einen erneuten Start der Bestrahlung der Wunde nach einem Therapieende durch einfaches und / oder versehentliches Drücken der Taste Start/Stopp zu verhindern, werden alle zu behandelnden Flächen nach einem Therapieende deselektiert. Die Taste Start/Stopp hat ferner in vorteilhafter Weise eine Art Pause-Funktion. Sofern die vorgegebenen Abstände eingehalten werden, kann die Therapie nach einem Stoppen bzw. auch nach einem automatischen Stopp wegen eines falschen Abstandes durch erneutes Drücken der Taste Start/Stopp wieder aufgenommen werden; der erneute Start erfolgt an der zuletzt bestrahlten Position, wobei die Bestrahlungszeit für diese Position noch einmal von vorne beginnt. Ggf. kann die Bestrahlungszeit für den erneuten Start für die genannte Position auch vorgegeben oder berechnet werden, insbesondere aus der verbliebenen Restzeit plus einer Pufferzeit, welche bevorzugt zumindest näherungsweise mit 5 Sekunden vorgegeben ist.

Die nachfolgend als Anforderungen und Kriterien für die verschiedenen Bestandteile oder Komponenten der Vorrichtung angegebenen Merkmale, Eigenschaften oder Funktionsweisen werden im Rahmen der Erfindung zusätzlich oder alternativ zu den vorangegangenen Erläuterungen, und zwar einzeln oder in zweckmäßiger Kombination in der erfindungsgemäßen Vorrichtung verwirklicht oder mit dem erfindungsgemäßen Verfahren durchgeführt.

### ANTRIEB

Mit drei Laserdioden soll eine Fläche von ca. 15x13cm bestrahlt werden können. Dazu ist es erforderlich, dass die Dioden durch einen Linearantrieb bewegt werden. Die Ansteuerung des Linearantriebs wird durch das Echtzeit-System erledigt.
Die beiden äußeren Dioden sind in einem starren Winkel von 20°(in Bezug zur Bestrahlungsebene bzw. zur mittleren Diode) angeordnet.

### FARBKAMERA

Das dargestellte Kamerabild soll eine Aufnahme der gesamten, zu bestrahlenden Fläche darstellen. Ist die Darstellung mit einer Aufnahme nicht möglich, so können entweder mehrere Kameras, oder eine Kamera mit einer Linearführung (eigene oder mit einer Laserführung) verwendet werden. Das aufgenommene Kamerabild muss vor der Darstellung am Display durch die Software am PC-System bearbeitet werden, um eine möglichst authentische Darstellung der Wunde zu erhalten. (Optische Kontrolle des aufgenommenen Bildes)

### ZUSÄTZLICHE LICHTQUELLE

Sind die Lichtverhältnisse wegen des geringen Abstandes zur Wunde nicht ausreichend, so ist eine Lichtquelle vorzusehen. Die Lichtquelle ist während der Dauer der Aufnahmen zu aktivieren (Scan). Während der Startbildschirm aktiv ist, wird die Lichtquelle nur eingeschaltet, wenn einer der Abstandssensoren einen Abstand <200 mm misst (damit Licht nur an ist, wenn mit dem Gerät gearbeitet wird, nicht sofort wenn es eingeschaltet ist). Beim Bestrahlen wird das Licht ausgeschaltet.

### ABSTANDSSENSOREN

Die Messung des Abstandes zwischen der Haut und der Laserdioden sollte 100mm (AKM 1 A4) betragen. Die Genauigkeit der Messung muss mindestens ±5 mm betragen. Der Abstandswert sollte im Servicemenü von Helbo änderbar sein. Zur Messung müssen geeignete Sensoren (Infrarot) verwendet werden. Pro Laserdiode ist ein Abstandssensor zu verwenden, welcher den Abstand im Zentrum der bestrahlten Fläche misst (bezogen auf die Längsachse des Geräts). Zusätzlich wird ein Sensor am Gehäuse benötigt. Die Einrichtung des Gerätes erfolgt durch den Sensor am Gehäuse und durch den Sensor an der mittleren Laserdiode. (Kontrolle und Vergleich des Messergebnisses mit dem tatsächlichen Abstand)

### DISPLAY

Für die Visualisierung der Kameraaufnahmen, Auswahl der zu bestrahlenden Flächen und zur Bedienung muss ein Touch Screen mit mindestens 8" eingesetzt werden.
Die Bedienung muss mit Einweghandschuhen möglich sein. Das Display und das Gehäuse müssen gegen Feuchtigkeitseintritt von oben geschützt sein. (Optische Kontrolle des Kamerabildes, Funktionsüberprüfung des Touch Screens)

### EINGABETASTEN

Der Schlüsselschalter unterbricht die gesamte Stromversorgung des Echtzeit-Systems und des PCSystems. Nach dem Einschalten über den Schlüsselschalter starten das Echtzeit- und das PC-System und der Startbildschirm wird angezeigt. Dadurch ist auch sichergestellt, dass die Bestrahlung nach einem Stromausfall nicht wieder automatisch startet. Wenn das Gerät eingeschaltet ist, bewirkt das Drücken einer Stand-By-Taste ein Abschalten des Displays und je nach technischer Machbarkeit und Sinnhaftigkeit auch des PCund 'Echtzeit-Systems (=Stand-By Modus). Dieser Modus wird durch das Leuchten einer roten LED (ev. direkt beim Taster) signalisiert. Einschalten aus dem Stand-By: durch erneutes Drücken der Stand-By-Taste (falls technisch möglich auch durch antippen des Touchscreens) - alle Systemkomponenten werden wieder hochgefahren - Anzeige des Startbildschirms. Die Betriebsbereitschaft wird durch eine grüne LED signalisiert (dafür kann die grüne LED verwendet) - rote Tasten-LED ist dann ausgeschaltet (oder grün).

Während der Bestrahlung oder im Pause-Modus ist die Stand-By-Taste wirkungslos.

Nach 20 Minuten Inaktivität (Zeit konfigurierbar) wird automatisch der Stand-By Zustand aktiviert.

Am Display und / oder Touch Screen sind folgende Bedienelement vorhanden:
- Weiter/Zurück:
- Wechsel zwischen Bildschirmen
- Scan:
   Startet die Aufnahme des Kamerabildes
- Start/Pause:
   Startet bzw. unterbricht die Bestrahlung der ausgewählten Felder
- Abbruch:
   Unterbricht die Therapie
- Alarmsignal-Inaktivschaltung ("Audio pausierend")

Es sind jeweils nur jene Bedienelemente auswählbar, welche laut Therapieablauf gerade aktiviert werden dürfen. Bedienelemente, welche aktivierbar sind, sind grafisch oder farblich von den nicht aktiven abzuheben (z.B. Umrandung).

Alle Bedienelemente sind gegen Flüssigkeitseintritt zu schützen.
(Bestätigen der Tasten und Funktionstest durchführen).

### NOT-AUS

Durch den Not-Aus Taster wird das Echtzeit-System (inkl. aller Antriebsmotoren und Laserdioden) gestoppt. Der Not-Aus Taster muss gemäß den Richtlinien (EN 60601-1-22) ausgeführt werden.

Die fernbedienbare Sicherheitseinrichtung wird wie ein Not-Aus Taster behandelt.
Wird der Not Aus betätigt, so wird die Spannungsversorgung folgender Komponenten unterbrochen:
- Linearantriebe
- Laserdioden
- Echtzeit-System

Aufgrund der hohen Stromaufnahme der Laserdioden erfolgt die Unterbrechung der Spannungsversorgung durch ein Relais.

Am Bildschirm wird eine Meldung ausgegeben, dass ein Not-Aus ausgelöst wurde: "Not-Aus oder externe Sicherheitsschaltung aktiviert. Bitte überprüfen Sie den Grund der Unterbrechung und starten Sie dann die Therapie neu."

Nachdem der Not-Aus Taster deaktiviert wurde, wird das System wieder initialisiert, die Meldung verschwindet (ohne Bestätigung durch Benutzer) und der Startbildschirm wird angezeigt. (Not Aus und Türkontaktschalter betätigen).

### EINGRIFFSCHUTZ

An der Unterseite des Gerätes ist ein Eingriffschutz vor zu sehen. Wird während der Bestrahlung in das Gerät hinein gegriffen, so muss die Laserstrahlung unterbrochen werden (Pause Modus).

Der Eingriffschutz wird über die Abstandssensoren realisiert. Wird während der kontinuierlichen Messung ein Abstand zu einem Objekt kleiner als 80 mm gemessen (Abstand Laserdioden-Austrittsöffnung - Haut, konfigurierbar), werden alle Antriebsmotoren und Laserdioden gestoppt. Dieser Abstandswert sollte im Servicemenü von Helbo änderbar sein. Ein entsprechender Warnhinweis wird am Display ausgegeben und das Programm in den Pause-Modus versetzt.

### LASERBETRIEBSANZEIGE

Die Laserbetriebsanzeige muss den Normen EN 60825, EN 60601-1-22 entsprechen und kann entweder am Display oder mit LEDs erfolgen. Für die Betriebsanzeige sind drei Farben zu verwenden:
- Grün:
   Laser Betriebsbereit - Schlüsselschalter aktiviert, PC- und Echtzeitsystem hochgefahren, Laserdioden haben Betriebstemperatur
- Gelb:
   Laser Emission
- Rot:
   Ein Fehler ist aufgetreten:
   □ Laserdiode defekt
   □ Sonstige kritische Fehler
Hinweis Laserschutzbrille: Rote Symbole und Hinweistexte nach Möglichkeit vermeiden! (Optische Kontrolle der Betriebsanzeige)

### BETRIEBSZEITEN SPEICHERUNG

Die Betriebszeiten der Laserdioden müssen dokumentiert werden.
Die gespeicherten Betriebszeiten sollten zurückgesetzt werden können. (Auslesen der Betriebszeiten nach abgelaufener Behandlung).

### STEUERUNGSMODUL

Das Steuerungsmodul "M1 B" umfasst die Steuerung und Positionierung der Laserdioden. Das Steuermodul "M1 B" wird in zwei Komponenten unterteilt:
- Echtzeit-System:
   Alle sicherheitskritischen Vorgänge, wie die Ansteuerung der Laserdioden, Abstandsmessung, Eingriffschutz, usw. werden durch diese Komponente erledigt. Die Steuerung muss echtzeitfähig sein.
- PC-System:
   Die Bildverarbeitung, Auswahl der zu bestrahlenden Bereiche, Betriebszeitenspeicherung und die Benutzerinteraktion erfolgt über das PC-System.

### SCHNITTSTELLE DER EMBEDDED KOMPONENTEN

Das PC-System und das Echtzeit-System kommunizieren über eine serielle Schnittstelle. Die Kommunikation ist durch ein Übertragungsprotokoll gesichert. Im Protokoll ist ein "heart-beat" zu definieren, der zyklisch gesendet werden muss. Antwortet einer der Komponenten nicht mehr, so muss eine Not Abschaltung eingeleitet werden. Am Display wird eine Fehlermeldung angezeigt. (Kommunikation des PC-Systems und Echtzeit-Systems unterbrechen, Fehler muss angezeigt werden, Behandlung wird abgebrochen.)

### LASERDIODEN STROMÜBERWACHUNG

Die Leistungsaufnahme der Laserdioden muss während der Bestrahlung überwacht werden. Weicht die Stromaufnahme um mehr als 20% ab, muss die Behandlung abgebrochen werden, da die Diode eventuell defekt ist.
Die Stromüberwachung erfolgt durch einen Ausgang des Laser-Steuergerätes.

### EMV, EXPLOSIONSSCHUTZ

Die EMV-Normen (EN 60601-1-2) müssen bei der Entwicklung der Hardware berücksichtigt werden, die Bestimmungen für den Explosionsschutz gemäß EN 60601-1 sind ebenfalls zu beachten.

### FERNBEDIENBARE SICHERHEITSEINRICHTUNG

Türkontaktschalter nach den Normen EN 60825, EN 60601-1-22.
Wird der Schalter betätigt (Kontakt geöffnet), so müssen die Laserdioden ausgeschaltet werden (Funktion wie Not Aus). Die elektronische Schaltung für diese Funktion hat die Prüfungen It. EN 60601-1 zu bestehen (Stromstoßfestigkeit).

### ECHTZEIT-SYSTEM

### AUFGABEN

Das Echtzeit-System ist verantwortlich für die
- Freigabe der Betriebswirtschaft
- Steuerung des Linearantriebes
- Abstandmessung
- Steuerung der Laserdioden

### FREIGABE DER BESTRAHLUNG

Nach dem Einrichten der Bestrahlungseinheit wird die gesamte Länge der Bestrahlungseinrichtung einmal abgefahren, um den Abstand an jeder Position zu messen (pro auswählbarer Fläche wird der minimale und maximale Abstand ermittelt und daraus ergibt sich, ob die Fläche insgesamt im gültigen Bereich liegt). Die gültigen Bereiche (8-12cm Abstand, diese Abstandswerte sollte im Servicemenü von Helbo änderbar sein) werden an das PC-System übermittelt. Eine Auswahl der Felder bei zu geringem Abstand nicht möglich.

### ABSTANDSMESSUNG

Die Abstandsmessung muss kontinuierlich durch das Echtzeit-System erfolgen. Die Sensoren für die Abstandsmessung sind am Echtzeit-System angeschlossen. Der Abstand eines jeden Sensors wird mehrmals pro Sekunde eingelesen und verarbeitet.

### ABSCHALTUNG BEI ZU GERINGEM ABSTAND

Ist der Abstand außerhalb des erlaubten Bereichs «80mm oder >120mm, die Abstandswerte sollten im Servicemenü von Helbo änderbar sein - ein eigenes Set an Grenzwerten ist vorzusehen (nicht jene vom Scan)) muss eine Not Abschaltung der Laserdioden erfolgen. Das Programm wird unterbrochen und eine Meldung an das PC-System gesendet. (Abstand verkleinern - Abschaltung muss erfolgen)

### START DER BESTRAHLUNG

Die Bestrahlung darf nur gestartet werden, wenn der Abstand zur Wunde für die zu bestrahlende Fläche im grünen Bereich ist.
(Bestrahlung darf bei zu geringem Abstand nicht gestartet werden - Veränderung des Abstandes nach Auswahl der Flächen).

### BESTRAHLUNG

Wird für eine Fläche die Bestrahlung gestartet, muss zuerst die Lasereinheit positioniert werden. Im Anschluss wird die Laserdiode für eine bestimmte Zeitdauer (40s) aktiviert. Die Dauer ist abhängig von der abgegebenen Leistung der Laserdiode und sollte im Servicemenü von Helbo änderbar sein.
Ist die Zeit für die zu bestrahlende Fläche abgelaufen, wird die Lasereinheit über der nächsten Fläche positioniert. Die Laserdioden sollten nicht bei jedem Schritt aus- und wieder eingeschaltet werden (wenn 2 Flächen hintereinander bestrahlt werden). Flächen, die nicht zur Bestrahlung ausgewählt sind, werden ausgelassen. ist keine weitere Fläche mehr vorhanden, wird die Bestrahlung ordnungsgemäß beendet. Eine Fläche entspricht in Längsrichtung 5 Bestrahlposition (dieser Wert und dadurch die Verfahrstrecke (6mm) sollten im Servicemenü von Helbo veränderbar sein).
Durch das Echtzeit-System dürfen nur Flächen bestrahlt werden, welche am PC-System ausgewählt wurden.
(Auswahl der Flächen und Kontrolle der bestrahlten Flächen).

### THERAPIEENDE

Sind alle Flächen bestrahlt worden, so werden die Laserdioden deaktiviert, das Ende der Therapie wird an das PC-System gemeldet.
(Ende der Therapie abwarten)

### ZEITÜBERSCHREITUNG

Eine Fläche darf nur eine bestimmte Zeit bestrahlt werden. Die Kontrolle der Zeit hat durch zwei von einander unabhängige Systeme zu erfolgen (EN 60601-1-22).
Einerseits wird die Zeit der Laserdiode durch das Echtzeit-System gemessen. Wird die maximale Zeit überschritten, wird die Diode abgeschalten und ein Fehler an das PC-System übermittelt. Andererseits wird die Einschaltdauer der Laserdiode durch eine geeignete Hardwareschaltung überwacht. Ist die Diode zu lange eingeschalten, wird die Laserdiode durch die Hardware deaktiviert_ Die maximale Einschaltdauer pro Position beträgt 120 Sekunden.

### PAUSE WÄHREND DER BESTRAHLUNG

Durch das PC-System kann eine Pause der Bestrahlung veranlasst werden. Erhält das EchtzeitSystem ein Pause-Kommando, so wird die Bestrahlung unterbrochen und die Laserdioden deaktiviert. Wird die Therapie fortgesetzt, wird die restliche Zeit um 5 Sekunden erhöht (Wert sollte im Servicemenü durch Helbo änderbar sein), und die Fläche wird weiter bestrahlt. (Pause auslösen, Bestrahlung wird korrekt fortgesetzt).

### SICHERHEITSFUNKTIONEN

Um Programmabstürze und Endlosschleifen vorzubeugen, wird ein Watchdog implementiert. Der Watchdog muss in zyklischen Abständen durch das Programm zurück gesetzt werden, ansonsten wird ein Reset durchgeführt.
Um sicher zu stellen, dass eine Fläche nicht zu lange bestrahlt wird, erfolgt eine automatische Abschaltung durch die Hardware, nachdem eine Laserdiode iänger als 120 Sekunden eingeschalten war.
Die Laserdioden Temperatur und der Diodenstrom wird kontinuierlich überwacht. Ist ein Wert außerhalb des gültigen Bereiches, wird abgeschalten um einen Defekt der Laserdioden zu verhindern. Wird der Türkontaktschalter oder Not-Aus-Schalter betätigt, so müssen die Laserdioden ausgeschaltet werden.
Ist die Kommunikation zwischen dem PC-System und dem Echtzeit-System unterbrochen, muss die Therapie abgebrochen werden.

### KÜHLUNG

Jede Laserdiode wird durch einen Lüfter gekühlt. Das Tachosignal des Lüfters muss überwacht werden. Fällt ein Lüfter aus, wird die Behandlung abgebrochen und ein Fehler an das PC-System gemeldet.

### BETRIEBSBEREITSCHAFT

Die Betriebsbereitschaft wird erst an das PC-System gemeldet, wenn alle Laserdioden Betriebstemperatur haben, und die Lüfter funktionieren.

### PC-System

- Das PC-System ist verantwortlich für:
- die Visualisierung der Kameraaufnahmen
- Darstellung der Abstandsmessung
- Starten und Stoppen der Bestrahlung
- Speicherung der Betriebszeiten

- Speicherung der zu bestrahlenden Flächen

### SCREENS / MELDUNGEN / HINWEISTÖNE

Bildschirmanzeigen:
1. Begrüßungsbildschirm (Optional): Aus, Weiter
2. Startbildschirm: erscheint nach dem Systemstart und dient zum Positionieren der Bestrahlungseinheit - das Live-Kamerabild wird angezeigt, überblendet mit den Abstandsanzeigen. Bedienelemente: Zurück (zum Begrüßungsbildschirm), Weiter (zum Therapiebildschirm)
3. Therapiebildschirm: Anzeige des Bestrahlungsrasters mit darunterliegendem Kamerabild, Miniaturanzeige der aktuellen Abstandsmessung, Bestrahlungsfortschritt. Bedienelemente: Zurück (zum Startbildschirm), Scan, Start/Pause, Abbruch
4. Service-Menü: Einstellungen, darf dem Anwender nicht zugänglich sein

Bei allen Bildschirmen ist ein Bedienelement Audio-Pausierend vorzusehen.

### Sprache:

Deutsch, Englisch -- Mehrsprachigkeit sollte vorgesehen werden, weitere Sprachen müssen mit geringem Aufwand implementierbar sein.
Servicemenü nur Englisch.

### Symbole:

alle Meldungen sollten durch Symbole unterstützt werden.

### Fehlermeldungen:

2 Fehlerklassen für Meldungen:
   a) Anwender- oder Therapiefehler - Fehlermeldung siehe jeweiliges REQ
   b) System- oder Gerätefehler, Aufleuchten der roten Fehler-LED, Fehlermeldung: "Gerätefehler xxx. Bitte starten Sie das Gerät neu. Falls der Fehler erneut auftritt rufen Sie bitte den technischen Support."

Hinweistöne: zu jeder Meldung sollte ein akustisches Alarmsignal abgegeben werden.

### ABSTANDSMESSUNG - GRAFISCHE DARSTELLUNG

Die Daten der Abstandsmessung des Echtzeit-Systems werden im PC-System verarbeitet, der Abstand wird farblich und grafisch am Display dargestellt.
Die Farben der Balken werden wie folgt definiert (Abstände im Service Menü konfigurierbar):
- dunkelgrün: Abstand vom !dealabstand (100mm) ist ±5mm (95mm-105mm)
- hellgrün: Abstand vom [dealabstand ist ±1 Omm (90mm-110mm)
- hellrot: Abstand vom Idealabstand ist ±20mm (80mm-120mm)
- dunkelrot: Abstand vom Idealabstand ist >20mm
(die Farbwiedergabe ist durch die normgerechte Laserschutzbrillen zu verifizieren!)

Die Abstandsanzeige wird auf 2 verschiedenen Eingabeschirmen dargestellt:
1. Startbildschirm: Einrichten der Bestrahlungseinheit - das Live-Kamerabild (unbearbeitet) wird angezeigt (Schlitten mit Kamera und Abstandssensor in jener Endposition, an der nicht der fixe Abstandssensor montiert ist), überblendet mit den Abstandsanzeigen. Die Abstandbalken werden dabei transparent dargestellt, nur jene Balken die den aktuell gemessenen Abstand repräsentieren werden vollfarbig dargestellt.
2. Therapiebildschirm: Miniaturanzeige der aktuellen Abstandsmessung (fixer Sensor und mittlerer Abstandssensor am Schlitten) (Optional)

Einrichten der Bestrahlungseinheit: Wird die Bestrahlungseinheit über einer großen Wunde platziert, so muss der Abstand an beiden Enden im grünen Bereich (=Abstand 9-11 cm, dieser Abstandswert sollte im Servicemenü von Helbo änderbar sein). liegen. Für kleinere Wunden ist es möglich, dass nur ein Ende der Bestrahlungseinrichtung im grünen Bereich liegt. Der Abstand beim Einrichten wird bei der mittleren Laserdiode gemessen. Beim Scan-Vorgang werden die gültigen Bereiche ermittelt (der Vorgang kann gestartet werden, wenn ein Ende der Bestrahlungseinrichtung im gültigen Bereich liegt.
(Vergleich der Daten aus dem Echtzeit-System und der Darstellung am Display).

### KAMERABILD, RASTER, BESTRAHLUNGSFLÄCHE

Das Kamerabild wird durch das PC-System verarbeitet und am Display dargestellt. Ein Raster wird über das Bild gelegt. Die Größe eines Raster-Feldes (im Bestrahlungsgebiet) beträgt: Länge (in Gerätelängsachse): 30mm (entspricht 5 Bestrahlungspositionen ä 6mm)
Breite: 45mm
Das Raster besteht aus insgesamt 5x3 Feldern, das Gesamte Bild deckt somit eine Fläche von 150mm x 105-135mm ab.
Die Größe eines Feldes am Bildschirm beträgt etwa die Hälfte der Originalgröße (15x23mm BxH) - jedenfalls so groß wie möglich.
(Darstellung am Display mit Wunde vergleichen, Auswahl der Felder nur möglich, wenn der Abstand in Ordnung ist).

### SCAN

Beim Betätigen der Scan-Taste werden die Bildinformationen eingelesen und der Abstand zur Wunde wird an jeder Bestrahlungsposition gemessen. Meldung: "Scan beendet. Bitte wählen Sie die zu bestrahlenden Felder aus."

Jene Flächen, die laut Scan nicht im gültigen Abstandsbereich liegen werden automatisch mit einem roten X hinterlegt.

Bestrahlungsflächen sollten nur ausgewählt werden, wenn der Abstand der Laserdiode zur Wunde im gültigen Bereich liegt (50-120mm, gemessen jeweils in der Mitte des Bestrahlungsfeldes pro Laserdiode, alle Abstandswerte dieses REQ sollten im Servicemenü von Helbo änderbar sein). Dadurch ist auch die Bestrahlung kleinerer Wunden möglich, falls der Abstand nicht über die gesamte Länge des Gerätes eingehalten werden kann.
Der Benutzer kann aber auch die ungültigen Teilflächen (X) zur Bestrahlung auswählen.

### START

Die Bestrahlung kann vom PC-System nur gestartet werden, wenn mindestens eine Fläche selektiert ist, und der Abstand zur Wunde im gültigen Bereich ist.

Vor dem Start der Bestrahlung erscheint noch eine Meldung "Therapie startet - Schutzbrille tragen!" (mit Brillen-Symbol) und ein Signalton. Die Bestrahlung wird nach 3 Sekunden Verzögerung gestartet, die Meldung erlischt selbsttätig nach 10 Sekunden (konfigurierbar).

Der Text der Schaltfläche "Start" wird in "Pause" geändert: (Start nur möglich, wenn mindestens eine Fläche selektiert ist).

### BESTRAHLUNG

Die Flächen, welche ausgewählt wurden, werden dem Echtzeit-System übermittelt. Jede ausgewählte Fläche wird für 40s bestrahlt. Diese Zeitdauer sollte im Servicemenü von Heibo änderbar sein. Eine Änderung der Flächen ist nicht mehr möglich, sobald das Programm gestartet wurde.
Die laufende Therapie und die bestrahlten Flächen werden am Display visualisiert, etwa durch einen wandernden Balken, welcher die Laserposition darstellt, Bereits bestrahlte Flächen sollten farblich abgehoben werden (anderer Rot-Ton).
Die Rest-Bestrahlungszeit ist anzuzeigen.
(Kontrolle der Laserposition und Vergleich mit einem ausgewählten Flächen).

### KONTINUIERLICHE ABSTANDSKONTROLLE

Wird durch das Echtzeit-System ein zu geringer oder zu großer Abstand einer Laserdiode (gilt für alle 3) gemeldet, so wird ein Hinweis am Display angezeigt und das Programm wird in den Pausemodus versetzt. Erst wenn der Abstand wieder im gültigen Bereich liegt (80mm bis 120mm, die Abstandswerte sollten im Servicemenü von Helbo änderbar sein), kann die Therapie fortgesetzt werden. Die Überwachung für den minimalen Abstand ist immer aktiviert, für den maximalen Abstand jedoch nur dann, wenn jene Fläche zur Bestrahlung ausgewählt wurde.

### THERAPIEENDE

Das Ende der Therapie wird durch das Echtzeit-System gemeldet, sobald alle Flächen bestrahlt wurden. Das PC-System gibt eine akustische und optische Meldung aus. Alle Flächen werden abgewählt um eine erneute Bestrahlung zu verhindern.
Für die Darstellung der Meldung ist ein Text am Display notwendig (eventuell Übersetzungen erforderlich).

Wurde während der Bestrahlung der minimale Abstand unterschritten (REQ 305), ist nach Quittieren der Meldung "Therapie beendet" zusätzlich ein Hinweis auf Hygienemaßnahmen am Display anzuzeigen, da der Patient ev. das Gerät berührt hat. Diese Meldung ist zu bestätigen und erscheint zusätzlich noch einmal vor Beginn der nächsten Behandlung (nach Einschalten oder Einschalten aus dem Standby).
Text: Patient hat möglicherweise das Gerät berührt - bitte besonders sorgfältig reinigen!

### ZEITÜBERSCHREITUNG

Wird durch das Echtzeit-System eine Zeitüberschreitung (maximale Einschaltdauer einer Laserdiode) gemeldet, wird ein Fehler am Display angezeigt, das Fortsetzen der laufenden Therapie ist nicht mehr möglich.
Für die Darstellung der Fehlermeldung ist ein Text am Display notwendig (eventuell Übersetzungen erforderlich): "Gerätefehler xxx. Bitte starten Sie das Gerät neu. Falls der Fehler erneut auftritt rufen Sie bitte den technischen Support."

Hinweis Laserschutzbrille: Rote Symbole und Hinweistexte nach Möglichkeit vermeiden!

### PAUSE WÄHREND DER BESTRAHLUNG

Über eine Taste am Touch Screen kann die aktuelle Therapie pausiert werden.

Der Text der Schaltfläche "Pause" wird in "Start" geändert.
Der Balken der Fortschrittsanzeige blinkt.
Eine Hinweismeldung wird über das Raster gefegt: "Bitte stellen Sie sicher, dass der bestrahlte

Körperteil noch richtig positioniert ist, ansonsten brechen Sie die Therapie ab."

Das PC-System wechselt ebenfalls in den Pause-Modus, wenn der Abstand zur Wunde während der Bestrahlung nicht mehr im gültigen Bereich liegt

Wenn sich das Gerät im Pause-Modus befindet, gibt es 2 Möglichkeiten um fortzufahren: Mit Start/Pause-Taste Behandlung fortsetzen (Abstände müssen wieder passen) Abbruch-Taste: (falls Abstände nicht mehr passen oder aus sonstigen Gründen, z. B. Patient hat sich zu viel bewegt)
(Therapie unterbrechen und fortsetzen.)

### ABBRUCH

Über eine Taste am Touch Screen kann die aktuelle Therapie abgebrochen werden. Folgende Szenarien sind möglich:
1. Während Bestrahlung oder
2. im Pause-Modus: Abfrage "Therapie läuft. Wollen Sie wirklich abbrechen?", bei Abbruch: "Therapie abgebrochen. Ausgewählter Bereich wurde nicht vollständig bestrahlt!"
3. Während Anzeige des Therapiebildschirms (gleiche Funktion wie "Zurück")

Nach Abbruch wird Gerät in den Ausgangszustand (Startbildschirm) zurückversetzt. (Therapie unterbrechen und fortsetzen).

### BETRIEBSZEITEN

Folgende Daten werden gespeichert:
- Betriebszeit in Sekunden pro Laserdioden
- Beginn einer Behandlung
- Bestrahlte Felder pro Behandlung
- Fehler
- Gesamtbetriebszeit

Die Daten können am Display in einem Service-Menü angezeigt werden. Als Sprache für dieses Menü wird Englisch verwendet.
In das Service-Menü gelangt man durch eine spezielle Kombination am Touch Screen.

### SICHERHEITSFUNKTIONEN

Die Kommunikation zum Echtzeit-System ist durch ein Übertragungsprotokoll gesichert. Fällt die Verbindung aus oder antwortet das Echtzeit-System nicht mehr, wird ein Fehler am Display angezeigt.

### ALLGEMEINE ANFORDERUNGEN

Bei der Entwicklung des Gehäuses sind die Normen EN 60601-1, EN 60625-1 zu beachten. Weiters ist die Gebrauchstauglichkeit für medizintechnisch elektrische Geräte nach EN 606011-6 zu verifizieren und validieren.
Das Gesamtgewicht der Bestrahlungseinheit soll 13 kg nicht überschreiten. Daher sind leichte Materialien (z.B. Aluminium) zu verwenden. In der Bestrahlungseinheit sollen daher auch nur Komponenten eingebaut werden, welche unbedingt erforderlich sind. Das PC-System soll aufgrund des Gewichtes daher mit Ausnahme des Touch Screens = im Gerätewage eingebaut werden. Das Gehäuse soll leicht zu reinigen sein, Nischen und Ritzen sind zu vermeiden.

### GEHÄUSE

Im Gehäuse der Bestrahlungseinheit müssen folgende Komponenten integriert werden:
- Linearantrieb und Führungsrahmen
- Laserdioden
- Steuergeräte der Laserdioden
- Hardware des Echtzeit-Systems
- Touch Screen, PC
- Laserbetriebsanzeige
- Stand-By Taste

Das Gehäuse muss gegen Flüssigkeitseintritt von oben geschützt sein. Lüftungsschlitze dürfen nicht auf die Wunde des Patienten oder zum Patienten gerichtet sein. Auf der Unterseite ist eine Abdeckung (durchsichtige Polycarbonat (PC)-Platte, gebogen (r=240mm) entlang der Gerätelängsachse, 2mm stark) angebracht, um Beschädigungen zu vermeiden.

Seitlich am Gehäuse sind Griffe zur Positionierung der Bestrahlungseinheit erforderlich. Die Bestrahlungseinheit wird am Gelenkarm montiert. Die Aufnahme erfolgt über die VESA 75 Halterung, und ist so auszuführen, dass eine Rotationsbewegung der Bestrahlungseinheit möglich ist.
Die Größe des Gehäuses ist durch die Komponenten, welche eingebaut werden, und durch die Größe des Linearantriebes und der seitlichen Führung vorgegeben.

Der maximale Gehäuseableitstrom von 0,5 mA laut EN 60601-1 muss eingehalten werden. Die verwendeten Materialien sollen ebenfalls zur EMV Schirmung des Systems beitragen. Zum Schutz der Elektronik muss das Gehäuse gegen Flüssigkeitseintritt geschützt sein.

### LINEARFÜHRUNG

Die Laserdioden müssen auf einer Länge von 15 cm bewegt werden können. Dafür ist ein geeigneter Antrieb wie z.B. ein Schrittmotor mit einem Inkrementalgeber zur Positionsbestimmung zu verwenden.
Die Laserdioden müssen so montiert werden, dass Stöße abgefangen werden.

### FÜHRUNG DER SEITLICHEN DIODEN

Die seitlichen Laserdioden sind starr - in einem definierten Abstand - mit der zentralen Diode verbunden. Sie sind in einem Winkel von 20° nach innen geschwenkt.

**Bezugszeichen**

| | | | |
|---|---|---|---|
| 2 | Bestrahlungseinheit | 59 | Start-/Stopp-Taste |
| 4 | Energieversorgungseinheit | 60 | Wunde |
| 6 | Positioniereinrichtung / Gelenkarm / Positionierarm | 62 | Unterschenkel / Bein |
| | | 64/65 | Pfeil |
| 8 | Gerätewagen | 66 | Block |
| 10 | Lichtquelle / Laserdiode / Lasereinheit | 68,69 | Abstandssensor |
| 12 | Führungseinrichtung / Führungsschiene / Linearführung | 70,71 | Pfeil |
| | | 72,73 | Berührungspunkt |
| 14 | Bestrahlungsposition / Bestrahlungsfeld | 74 | grau unterlegte Fläche |
| 16 | Pfeil / Bewegungsrichtung | 76 | gekreuzt/schraffierte Fläche |
| 17 | Rahmen / Schlitten | | |
| 18 | Gehäuse | | |
| 19 | Antriebseinheit | | |
| 20 | Display | | |
| 22 | Kugelgelenk | | |
| 23 | Handgriff | | |
| 24 | Lasersystem | | |
| 26 | Linearführung | | |
| 28 | Kamera | | |
| 30 | Oberfläche / Linie | | |
| 32 | Laserdiode | | |
| 34 | Kühlkörper | | |
| 36 | Optik | | |
| 38 | Linie / Schutzscheibe | | |
| 40 | Bedieneinheit | | |
| 42 | Live-Kamera-Bild | | |
| 44 | Wundfläche | | |
| 46 | Raster | | |
| 47-49 | Bestrahlungsfeld | | |
| 50 | Mausklick | | |
| 51 | Markierung / Kreuz | | |
| 52,53 | Abstandsanzeige | | |
| 54,55 | Symbol / Balken | | |
| 56 | Spitze von 54, 55 | | |
| 58 | Scan-Taste | | |

## Patentansprüche

1. Vorrichtung für die photodynamische Therapie und / oder zur Abtötung oder Reduktion von Mikroorganismen, enthaltend eine Bestrahlungseinheit (2) mit wenigstens einer Lichtquelle (10.1, 10.2, 10.3), mittels welcher ein auf ein zu therapierendes Wundareal (44) aufgebrachter Photosensitizer durch Bestrahlung aktiviert wird, ferner enthaltend eine in der Bestrahlungseinheit (2) angeordnete Kamera (28) zur Erfassung von Bildern der Wunde sowie eine Positioniereinrichtung (6), mittels welcher die Bestrahlungseinheit (2) zum Wundareal (44) ausrichtbar ist, sowie ein Display (20), auf welchem das mittels der Kamera (28) erfasste Bild und ein diesem überlagertes eingeblendetes Raster (46) und Markierungsmittel (51) darstellbar sind, wobei die Bestrahlungseinheit (2) eine Führungseinrichtung mit einer Führungsschiene (12) und einen Rahmen oder Schlitten (17), auf welchem die Lichtquelle oder Lichtquellen (10.1, 10.2, 10.3) angeordnet sind, und ferner einer Antriebseinheit (19) enthält, **dadurch gekennzeichnet, dass** die Antriebseinheit (19) dazu ausgebildet ist, die Lichtquelle oder Lichtquellen (10.1, 10.2, 10.3) in einer Bewegungsrichtung (16) zu bewegen und sequentiell auf wenigstens zwei unterschiedliche Bestrahlungspositionen (14) des Wundareals (44) zu positionieren,
wobei in den Bestrahlungspositionen (14) jeweils Teilflächen des Wundareals (44) als Bestrahlungsfelder (47 bis 49) bestrahlbar sind,
dass das eingeblendete Raster (46) den Bestrahlungspositionen (14) der Lichtquelle oder Lichtquellen (10.1, 10.2, 10.3) entspricht
und dass das Display (20) derart ausgebildet ist, dass die mittels der Lichtquellen (10.1, 10.2, 10.3) zu bestrahlenden Bestrahlungsfelder (47 bis 49) mit den Markierungsmitteln (51) im Display (20) markierbar sind.

2. Vorrichtung nach Anspruch 1, **dadurch gekennzeichnet, dass** die Führungseinrichtung als eine Linearführung mit der Führungsschiene (12) ausgebildet ist.

3. Vorrichtung nach Anspruch 1 oder 2, **dadurch gekennzeichnet, dass** die Lichtquelle oder Lichtquellen (10.1, 10.2, 10.3) als Laserdioden ausgebildet sind und / oder auf einer Lasereinheit (10) angeordnet sind.

4. Vorrichtung nach einem der Ansprüche 1 bis 3, **dadurch gekennzeichnet, dass** wenigstens zwei Lichtquellen (10.1, 10.2, 10.3) bezogen auf die Bewegungsrichtung (16) hintereinander oder quer zu dieser angeordnet sind, wobei bevorzugt drei Lichtquellen (10.1, 10.2, 10.3) quer zur Bewegungsrichtung (16) angeordnet sind.

5. Vorrichtung nach einem der Ansprüche 1 bis 4, **dadurch gekennzeichnet, dass** das Display (20) zusammen mit Eingabetasten (58, 59) in einer Bedieneinheit (40) angeordnet sind.

6. Vorrichtung nach einem der Ansprüche 1 bis 5, **dadurch gekennzeichnet, dass** die Kamera (28) auf dem Schlitten (17), gemeinsam mit der wenigstens einer Lichtquelle (10.1, 10.2, 10.3) angeordnet und bewegbar ist.

7. Vorrichtung nach einem der Ansprüche 1 bis 6, **dadurch gekennzeichnet, dass** beim Bewegen der Lichtquelle oder Lichtquellen (10) entlang der Bewegungsrichtung (16) jene nur für die im Display (20) markierten Bestrahlungspositionen (14) oder Bestrahlungsfelder (A bis F) einschaltbar sind.

8. Vorrichtung nach einem der Ansprüche 1 bis 7, **dadurch gekennzeichnet, dass** die Lichtquelle oder Lichtquellen (10.1, 10.2, 10.3) mittels der Führungseinrichtung und der Antriebseinheit (19) entsprechend den, in das Display (20) eingeblendeten, Raster (46) derart sequentiell positionierbar sind, dass sämtliche in Bewegungsrichtung (16) und quer zu dieser angeordnete Bestrahlungsfelder entsprechend dem Kamerabild des Wundareals (44) bestrahlbar sind.

9. Vorrichtung nach einem der Ansprüche 1 bis 8, **dadurch gekennzeichnet, dass** wenigstens eine Lichtquelle (10.2, 10.3) des Lasersystems zu einer anderen Lichtquellen (10.1) schwenkbar oder in einem vorgegebenen Winkel geschwenkt auf dem System (24) angeordnet ist.

## Claims

1. An apparatus for photodynamic therapy and/or for destroying or reducing microorganisms, said apparatus containing an irradiation unit (2) with at least one light source (10.1, 10.2, 10.3), by means of which a photosensitizer applied to a wound site (44) to be treated is activated by radiation, further containing a camera (28), arranged in the irradiation unit (2), for recording images of the wound, and also a positioning device (6), by means of which the irradiation unit (2) can be aligned with the wound site (44), and also containing a display (20), on which the image recorded by means of the camera (28) and a superimposed, blended-in grid (46) and marking means (51) can be presented, the irradiation unit (2) containing a guide device with a guide rail (12) and a frame or carriage (17), on which the light source or light sources (10.1, 10.2, 10.3) are arranged, and further containing a drive unit (19), **characterised in that** the drive unit (19) is designed to move the light source or light sources (10.1, 10.2, 10.3) in a direction of movement (16) and to position said light source or light sources sequentially in at least two different irradiation positions (14) of the wound site (44),
it being possible to irradiate sub-areas of the wound site (44) as irradiation fields (47 to 49) in each of the irradiation positions (14),
**in that** the blended-in grid (46) corresponds to the irradiation positions (14) of the light source or light sources (10.1, 10.2, 10.3),
and **in that** the display (20) is designed in such a way that the irradiation fields (47 to 49) to be irradiated by means of the light sources (10.1, 10.2, 10.3) can be marked by the marking means (51) in the display (20).

2. The apparatus according to Claim 1, **characterised in that** the guide device is formed as a linear guide with the guide rail (12).

3. The apparatus according to Claim 1 or 2, **characterised in that** the light source or light sources (10.1, 10.2, 10.3) are formed as laser diodes and/or are arranged on a laser unit (10).

4. The apparatus according to one of Claims 1 to 3, **characterised in that** at least two light sources (10.1, 10.2, 10.3) are arranged in succession based on the direction of movement (16) or transversely with respect thereto, three light sources (10.1, 10.2, 10.3) preferably being arranged transversely with respect to the direction of movement (16).

5. The apparatus according to one of Claims 1 to 4, **characterised in that** the display (20) together with input keys (58, 59) is arranged in an operating unit (40).

6. The apparatus according to one of Claims 1 to 5, **characterised in that** the camera (28) is arranged and is movable on the carriage (17), jointly with the at least one light source (10.1, 10.2, 10.3).

7. The apparatus according to one of Claims 1 to 6, **characterised in that**, as the light source or light sources (10) is/are moved along the direction of movement (16), it/they can only be switched on for the irradiation positions (14) or irradiation fields (A to F) marked in the display (20).

8. The apparatus according to one of Claims 1 to 7, **characterised in that** the light source or light sources (10.1, 10.2, 10.3) can be positioned sequentially by means of the guide device and the drive unit (19) in accordance with the grid (46) blended into the display (20), in such a way that all irradiation fields arranged in the direction of movement (16) and transversely with respect thereto can be irradiated in accordance with the camera image of the wound site (44).

9. The apparatus according to one of Claims 1 to 8, **characterised in that** at least one light source (10.2, 10.3) of the laser system is arranged so as to be pivotable with respect to another light source (10.1) or is arranged on the system (24) pivoted at a predefined angle.

## Revendications

1. Dispositif de photochimiothérapie et/ou de destruction ou de réduction de microorganismes, comprenant une unité d'irradiation (2) avec au moins une source lumineuse (10.1, 10.2, 10.3) au moyen de laquelle un photosensibiliseur appliqué sur une surface de plaie (44) à traiter est activé par irradiation, comprenant en outre une caméra (28) disposée dans l'unité d'irradiation (2) pour saisir des images de la plaie ainsi qu'un dispositif de positionnement (6) au moyen duquel l'unité d'irradiation (2) peut être alignée sur la surface de plaie (44), ainsi qu'un écran (20) sur lequel l'image saisie via la caméra (28) et un quadrillage (46) inséré superposé à celle-ci et des moyens de marquage (51) peuvent être représentés, sachant que l'unité d'irradiation (2) contient un dispositif de guidage avec un rail de guidage (12) et un cadre ou un chariot (17) sur lequel la ou les source(s) lumineuse(s) (10.1, 10.2, 10.3) est/sont placée(s) et en outre une unité d'entraînement (19), **caractérisé en ce que** l'unité d'entraînement (19) est conçue pour déplacer la ou les source(s) lumineuse(s) (10.1, 10.2, 10.3) dans une direction de mouvement (16) et pour les positionner de manière séquentielle sur au moins deux positions d'irradiation (14) différentes de la surface de plaie (44),
sachant que dans les positions d'irradiation (14), des surfaces partielles respectives de la surface de plaie (44) peuvent être irradiées en tant que champs d'irradiation (47 à 49),
que le quadrillage (46) inséré correspond aux positions d'irradiation (14) de la ou des source(s) lumineuse(s) (10.1, 10.2, 10.3)
et que l'écran (20) est ainsi conçu que les champs d'irradiation (47 à 49) à irradier via les sources lumineuses (10.1, 10.2, 10.3) peuvent être marqués à l'écran (20) avec les moyens de marquage (51).

2. Dispositif selon la revendication 1, **caractérisé en ce que** le dispositif de guidage est conçu en tant qu'un guidage linéaire avec le rail de guidage (12).

3. Dispositif selon la revendication 1 ou 2, **caractérisé en ce que** la ou les source(s) lumineuse(s) (10.1, 10.2, 10.3) sont formées en tant que diodes laser et/ou est/sont disposée(s) sur une unité laser (10).

4. Dispositif selon l'une des revendications 1 à 3, **caractérisé en ce qu'**au moins deux sources lumineuses (10.1, 10.2, 10.3), rapporté à la direction de mouvement (16), sont placées l'une derrière l'autre ou transversalement à celle-ci, sachant que de préférence trois sources lumineuses (10.1, 10.2, 10.3) sont disposées transversalement à la direction de mouvement (16).

5. Dispositif selon l'une des revendications 1 à 4, **caractérisé en ce que** l'écran (20) est disposé dans une unité de commande (40) conjointement avec des touches de saisie (58, 59).

6. Dispositif selon l'une des revendications 1 à 5, **caractérisé en ce que** la caméra (28) est disposée conjointement avec l'au moins une source lumineuse (10.1, 10.2, 10.3) sur le chariot (17) et est mobile.

7. Dispositif selon l'une des revendications 1 à 6, **caractérisé en ce que** lorsque l'on déplace la ou les source(s) lumineuse(s) (10) le long de la direction de mouvement (16), seule(s) celle(s) pour les positions d'irradiation (14) ou les champs d'irradiation (A à F) marqués à l'écran (20) sont déclenchées.

8. Dispositif selon l'une des revendications 1 à 7, **caractérisé en ce que** la ou les source(s) lumineuse(s) (10.1, 10.2, 10.3) peuvent être positionnées de manière séquentielle via le dispositif de guidage et le dispositif d'entraînement (19), conformément au quadrillage (46) inséré sur l'écran (20), que l'ensemble des champs d'irradiation disposés dans la direction de mouvement (16) et transversalement à celle-ci peuvent être irradiés conformément à l'image de la caméra de la surface de plaie (44).

9. Dispositif selon l'une des revendications 1 à 8, **caractérisé en ce qu'**au moins une source lumineuse (10.2, 10.3) du système laser est disposée en étant pivotable par rapport à une autre source lumineuse (10.1) ou basculée sur le système (24) dans un angle prédéfini.
